(19) Europäisches Patentamt
European Patent Office
Office européen des brevets

(11) EP 2 004 755 B1

(12) # FASCICULE DE BREVET EUROPEEN

(45) Date de publication et mention de la délivrance du brevet:
**03.08.2011 Bulletin 2011/31**

(21) Numéro de dépôt: **07731811.1**

(22) Date de dépôt: **23.03.2007**

(51) Int Cl.:
***C09B 23/14*** (2006.01) ***C09B 49/12*** (2006.01)
***A61Q 5/08*** (2006.01) ***A61Q 5/06*** (2006.01)

(86) Numéro de dépôt international:
**PCT/FR2007/050999**

(87) Numéro de publication internationale:
**WO 2007/110533 (04.10.2007 Gazette 2007/40)**

(54) **COMPOSITION DE TEINTURE COMPRENANT UN COLORANT FLUORESCENT THIOL/DISULFURE, A CHARGE CATIONIQUE EXTERNE ET CHAINE ALKYLENE INTERROMPUE, PROCEDE D'ECLAIRCISSEMENT DES MATIERES KERATINIQUES A PARTIR DE CE COLORANT**

FÄRBEZUSAMMENSETZUNG MIT EINEM THIOL/DISULFID- LEUCHTFARBSTOFF MIT EINER EXTERNEN KATIONISCHEN LADUNG UND EINER UNTERBROCHENEN ALKYLENKETTE SOWIE VERFAHREN ZUR AUFHELLUNG VON KERATINMATERIAL MIT DIESEM FARBSTOFF

DYEING COMPOSITION CONTAINING A THIOL/DISULPHIDE FLUORESCENT COLORANT HAVING AN EXTERNAL CATIONIC CHARGE AND AN INTERRUPTED ALKYLENE CHAIN, AND METHOD FOR LIGHTENING KERATIN MATERIALS USING SAID COLORANT

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **24.03.2006 FR 0651035**
**19.04.2006 US 792941 P**
**05.02.2007 FR 0753065**
**15.02.2007 US 901319 P**

(43) Date de publication de la demande:
**24.12.2008 Bulletin 2008/52**

(73) Titulaire: **L'Oréal**
**75008 Paris (FR)**

(72) Inventeurs:
- **GREAVES, Andrew**
  **F-77144 Montevrain (FR)**
- **DAUBRESSE, Nicolas**
  **F-78170 La Celles St Cloud (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:

| | |
|---|---|
| **EP-A- 1 647 580** | **WO-A-03/028685** |
| **WO-A-2005/004822** | **WO-A-2006/134043** |
| **WO-A-2006/136617** | **WO-A-2007/025889** |
| **WO-A2-2004/091473** | **WO-A2-2005/097051** |
| **GB-A- 2 180 215** | **US-A- 2 904 385** |

- **LAL M ET AL: "Silica Nanobubbles Containing an Organic Dye in a Multilayered Organic/Inorganic Heterostructure with Enhanced Luminescence" CHEMISTRY OF MATERIALS, AMERICAN CHEMICAL SOCIETY, WASHINGTON, US, vol. 12, no. 9, 19 août 2000 (2000-08-19), pages 2632-2639, XP002390713 ISSN: 0897-4756**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention concerne la coloration de fibres kératiniques à l'aide de colorants fluorescents thiols/disulfures pyridinium à charge cationique externe et chaîne alkylène interrompue.

Il est connu de teindre les fibres kératiniques, notamment humaines, par une coloration directe. Le procédé classiquement utilisé en coloration directe consiste à appliquer sur les fibres kératiniques des colorants directs qui sont des molécules colorées et colorantes ayant une affinité pour les fibres, à les laisser diffuser, puis à rincer les fibres.

Les colorants directs qui sont classiquement utilisés sont par exemple des colorants du type nitrés benzéniques, des colorants anthraquinoniques, des nitropyridines, des colorants du type azoïque, xanthénique, acridinique, azinique ou triarylméthane.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Par ailleurs, la coloration des fibres kératiniques à partir de colorants directs classiques ne permet pas d'éclaircir de façon notable les fibres kératiniques.

L'éclaircissement de la couleur de fibres kératiniques, plus particulièrement foncées vers des nuances plus claires, en modifiant éventuellement la nuance de celles-ci, constitue une demande importante.

Classiquement, pour obtenir une coloration plus claire on met en oeuvre un procédé de décoloration chimique. Ce procédé consiste à traiter les matières kératiniques telles que les fibres kératiniques, notamment les cheveux, par un système oxydant fort, généralement constitué par du peroxyde d'hydrogène associé ou non à des persels, le plus souvent en milieu alcalin.

Ce système de décoloration présente l'inconvénient de dégrader des matières kératiniques, en particulier les fibres kératiniques, notamment humaines telles que les cheveux et d'altérer leurs propriétés cosmétiques. Les fibres ont en effet tendance à devenir rêches, plus difficilement démêlables et plus fragiles. Enfin, l'éclaircissement ou la décoloration de fibres kératiniques par des agents oxydants est incompatible avec les traitements de modification de la forme desdites fibres particulièrement dans les traitements de défrisage.

Une autre technique d'éclaircissement consiste à appliquer sur les cheveux foncés des colorants directs fluorescents. Cette technique décrite notamment dans les documents FR 2830189 et WO 2004/091473 permet de respecter la qualité de la fibre kératinique lors du traitement mais les colorants fluorescents employés ne présentent pas une résistance aux shampoings satisfaisante.

Pour augmenter la ténacité des colorations directes, il est connu de fixer des colorants directs par liaison covalente au cheveu. Par exemple, il est connu de faire réagir des colorants à groupements réactifs avec les résidus de cystine ou de cystéine très nombreux dans les fibres kératiniques voir par exemple Journal of the Society of Dyers and Colourists, Guise et Stapleton, 91, 259-264 (1975): Journal of Cosmetic Chemistry, 42, 1-17 (1991); CA 2024509;

De plus il est connu de protéger la ou les fonctions thiols contenues dans une molécule à greffer aux cheveux avant de les appliquer auxdits cheveux WO 99/51194. Cependant cette demande ne mentionne pas l'utilisation de colorants fluorescents permettant de colorer ou d'éclaircir des cheveux.

D'autres colorants disulfures connus pour la coloration des fibres kératiniques sont des dérivés disulfures de dérivés d'aminothiophénol. De tels colorants sont décrits par exemple dans le brevet FR 1156407. Ces colorants peuvent être utilisés dans des conditions relativement douces, en présence d'un milieu légèrement réducteur ou après un prétraitement réducteur du cheveu. Cependant, ces colorants peuvent occasionner des virages de couleur lors de l'application.

Enfin, le document WO 2005/097051 décrit des colorants disulfures aza-imidazoliums pour la coloration directe de fibres kératiniques.

Le but de la présente invention est de fournir de nouveaux systèmes de coloration de matières kératiniques en particulier les fibres kératiniques humaines, notamment les cheveux, qui ne présentent pas les inconvénients des procédés de décoloration existants. En particulier, un des buts de la présente invention est de fournir des systèmes de coloration directe permettant d'obtenir des effets éclaircissants notamment sur des fibres kératiniques naturellement ou artificiellement foncées, tenaces face à des shampooings successifs, qui ne dégradent pas les fibres kératiniques et qui n'altèrent pas leurs propriétés cosmétiques.

Ce but est atteint avec la présente invention qui a pour objet un procédé de coloration de matières kératiniques, en particulier les fibres kératiniques, notamment humaines telles que les cheveux, plus particulièrement les cheveux foncés, consistant à appliquer sur les matières kératiniques, une composition tinctoriale, comprenant dans un milieu cosmétique approprié, au moins un colorant fluorescent thiol pyridinium à charge cationique externe choisi parmi les colorants de formule (I) ou (II) suivantes :

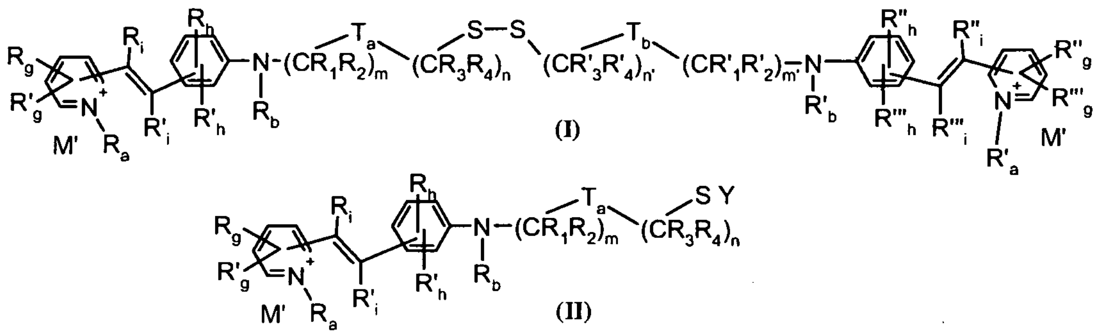

(I)

(II)

leurs sels d'acide organique ou minéral, isomères optiques, isomères géométriques, et les solvates tels que hydrates ; formule (I) dans laquelle :

➢ $R_a$ et $R'_a$, identiques ou différents, représentent un groupement aryl($C_1$-$C_4$)alkyle ou un groupement ($C_1$-$C_6$) alkyle éventuellement substitué par un groupement hydroxy ou amino, $C_1$-$C_4$ alkylamino, ($C_1$-$C_4$) dialkylamino, lesdits radicaux alkyle pouvant former avec l'atome d'azote qui les portent un hétérocycle comprenant de 5 à 7 chaînons, comprenant éventuellement un autre hétéroatome différent ou non de l'azote ; préférentiellement $R_a$ et $R'_a$ représentent un groupement ($C_1$-$C_3$) alkyle éventuellement substitué par un groupement hydroxy, ou un groupement benzyle ;

➢ $R_b$ et $R'_b$, identiques ou différents, représentent un atome d'hydrogène, un groupement aryl($C_1$-$C_4$)alkyle ou un groupement ($C_1$-$C_6$) alkyle éventuellement substitué; notamment $R_b$ et $R'_b$ représentent un atome d'hydrogène ou un groupement ($C_1$-$C_3$) alkyle ou benzyle ;

➢ $R_g$, $R'_g$, $R''_g$ et $R'''_g$, identiques ou différents, représentent un atome d'hydrogène, un groupement amino, ($C_1$-$C_4$) alkylamino, ($C_1$-$C_4$) dialkylamino, , trifluorométhyle, un radical acylamino, ($C_1$-$C_4$)alcoxy ($C_1$-$C_4$)alkylcarbonyloxy, ($C_1$-$C_4$)alcoxycarbonyle, ($C_1$-$C_4$)alkylcarbonylamino, ($C_1$-$C_4$)alkylsulfonylamino, un radical ($C_1$-$C_3$)alkyle;

➢ $R_h$, $R'_h$, $R''_h$ et $R'''_h$, identiques ou différents, représentent un atome d'hydrogène, un atome d'halogène, un groupement dialkylamino ($C_1$-$C_4$), ($C_1$-$C_4$)alkylcarbonylamino, un radical acylamino, ($C_1$-$C_4$)alkylsulfonylamino, ou un radical ($C_1$-$C_4$) alkyle ; particulièrement $R_h$, $R'_h$, $R''_h$ et $R'''_h$, représentent un atome d'hydrogène, ou un groupement ($C_1$-$C_3$)alkyle ;

➢ ou alors deux groupements $R_g$ et $R'_g$ ; $R''_g$ et $R'''_g$; $R_h$, et $R'_h$ ; $R''_h$ et $R'''_h$ portés par deux atomes de carbone adjacents, forment ensembles un cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, ($C_1$-C4)alkylamino, ($C_1$-$C_4$)dialkylamino, cyano, carboxy, hydroxy, trifluorométhyle, acylamino, alcoxy en $C_1$-$C_4$, (poly)hydroxyalcoxy en $C_2$-$C_4$, ($C_1$-$C_4$)alkylcarbonyloxy ($C_1$-$C_4$)alcoxycarbonyle, ($C_1$-$C_4$)alkylcarbonylamino, un radical acylamino, carbamoyle , ($C_1$-$C_4$)alkylsulfonyl-amino, un radical aminosulfonyle, ou un radical ($C_1$-$C_{16}$)alkyle éventuellement substitué par un groupement choisi parmi ($C_1$-$C_{12}$)alcoxy, hydroxy, cyano, carboxy, amino, ($C_1$-$C_4$)alkylamino et ($C_1$-$C_4$)dialkylamino, ou alors les deux radicaux alkyle portés par l'atome d'azote du groupement amino forment un hétérocycle comprenant de 5 à 7 chaînons et comprenant éventuellement un autre hétéroatome identique ou différent à celui de l'atome d'azote ; particulièrement $R_g$ et $R'_g$; $R''_g$ et $R'''_9$ forment ensemble un groupement benzo ;

➢ $R_i$, $R'_i$, $R''_j$ et $R'''_i$, identiques ou différents, représentent un atome d'hydrogène, ou un groupement ($C_1$-$C_4$)alkyle; particulièrement un atome d'hydrogène ;

➢ $R_1$, $R_2$, $R_3$, $R_4$, $R'_1$, $R'_2$, $R'_3$ et $R'_4$, identiques ou différents, représentent un atome d'hydrogène ou un groupement ($C_1$-$C_4$) alkyle, ($C_1$-$C_{12}$)alcoxy, hydroxy, cyano, carboxy, (di)($C_1$-$C_4$)(alkyl)amino, lesdits radicaux alkyle pouvant former avec l'atome d'azote qui les portent un hétérocycle comprenant de 5 à 7 chaînons, comprenant éventuellement un autre hétéroatome différent ou non de l'azote ; notamment $R_1$, $R_2$, $R_3$ et $R_4$ sont des atomes d'hydrogène ou un groupement (di)($C_1$-$C_4$)(alkyl)amino, préférentiellement $R_1$, $R_2$, $R'_1$ et $R'_2$ représentent un atome d'hydrogène et $R_3$, $R_4$, $R'_3$ ou $R'_4$ représentent un atome d'hydrogène ou un groupement (di)($C_1$-$C_4$)(alkyl)amino ; le groupement (di)($C_1$-$C_4$)(alkyl)amino se trouvant particulièrement en $\alpha$ de la liaison $T_a$ ou $T_b$ lorsque n ou n' est supérieur à 1; particulièrement $R_3$, $R_4$, $R'_3$ et $R'_4$, représentent un atome d'hydrogène ou un groupement amino et un seul des groupements $R_3$, $R_4$ et/ou un seul des groupements $R'_3$ et $R'_4$ ne peut représenter qu'un groupement amino, ledit groupement amino se trouvant préférentiellement en $\alpha$ de la liaison $T_a$ et/ou $T_b$ lorsque n ou n' est supérieur à 1 ; plus particulièrement $R_1$, $R_2$, $R_3$, $R_4$, $R'_1$, $R'_2$, $R'_3$ et $R'_4$, représentent un atome d'hydrogène ;

➢ $T_a$ et $T_b$, identiques ou différents, représentent la combinaisons des radicaux choisis parmi -$SO_2$-, -O-, -S-, -N (R)-, -CO-, avec R, représente un atome d'hydrogène, un radical alkyle en $C_1$-$C_4$, hydroxyalkyle en $C_1$-$C_4$; ou un

aryl($C_1$-$C_4$)alkyle, notamment représentent un groupement choisi parmi -C(O)-N(R)-, -N(R)-C(O)- ;

➢ m, m', n et n', identiques ou différents, représentent un entier compris inclusivement entre 0 et 6 avec m+n et m'+n', identiques ou différents, représentent un entier compris inclusivement entre 1 et 10 ; particulièrement la somme m+n=m'+n' est un entier compris inclusivement entre 4 et 6 ;

➢ M' représentant un contre-ion anionique ; et

➢ Y représente : i) un atome d'hydrogène ; ii) un métal alcalin ; iii) un métal alcalino-terreux ; iv) un groupement ammonium : $N^+R^\alpha R^\beta R^\gamma R^\delta$ ou un groupement phosphonium : $P^+R^\alpha R^\beta R^\gamma R^\delta$ avec $R^\alpha$, $R^\beta$, $R^\gamma$ et $R^\delta$, identiques ou différents, représentant un atome d'hydrogène ou un groupe ($C_1$-$C_4$)alkyle ; ou v) un groupement protecteur de fonction thiol ;

étant entendu que lorsque le composé de formule (I) ou (II) contient d'autres parties cationiques, il se trouve associé à un ou plusieurs contre-ions anioniques permettant d'atteindre l'électroneutralité de la formule (I) ou (II).

Un autre objet de l'invention est une composition tinctoriale comprenant, dans un milieu cosmétique approprié au moins un colorant fluorescent choisi parmi les colorants de formule (I) ou (II) tels que définis précédemment, et éventuellement un agent réducteur.

L'invention a aussi pour objet de nouveaux colorants fluorescents de formule (I) ou (II) tels que définis précédemment.

Le procédé de coloration selon l'invention permet de colorer de façon visible les matières kératiniques foncées, en particulier les fibres kératiniques humaines foncées, notamment les cheveux foncés.

De plus le procédé de l'invention permet d'obtenir une coloration des matières kératiniques, en particulier les fibres kératiniques humaines, notamment les cheveux, sans dégrader ladite matière, rémanente vis-à-vis de shampooings, des agressions courantes (soleil, transpiration), et des autres traitements capillaires. Le procédé de l'invention permet également d'obtenir un éclaircissement des matières kératiniques telles que les fibres kératiniques particulièrement les fibres kératiniques foncées et plus particulièrement les cheveux foncés.

Au sens de l'invention, on entend par matière kératinique foncée celle qui présente une luminescence L* chiffrée dans le système C.I.E.L*a*b*, inférieure ou égale à 45 et de préférence inférieure ou égale à 40, sachant par ailleurs que L*=0 équivaut au noir et L*=100 au blanc.

Au sens de l'invention, on entend par cheveux naturellement ou artificiellement foncés, des cheveux dont la hauteur de ton est inférieure ou égale à 6 (blond foncé) et de préférence inférieure ou égale à 4 (châtain).

L'éclaircissement des cheveux est évalué par la variation de « hauteur de ton » avant et après application du composé de formule (I) ou (II).

La notion « ton » repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Science des traitements capillaires » de Chartes ZVIAK 1988, Ed. Masson, p. 215 et 278.

Les hauteurs de ton s'échelonnent de 1 (noir) à 10 (blond très clair), une unité correspondant à un ton ; plus le chiffre est élevé et plus la nuance est claire.

Un cheveu coloré artificiellement est un cheveu dont la couleur a été modifiée par un traitement de coloration par exemple une coloration avec des colorants directs ou des colorants d'oxydation.

De préférence, la composition doit après application sur des cheveux, par exemple châtains, amener aux résultats ci-dessous.

- On s'intéresse aux performances de réflectance des cheveux lorsqu'on les irradie avec de la lumière visible dans la gamme de longueurs d'onde allant de 400 à 700 nanomètres.
- On compare alors les courbes de réflectance en fonction de la longueur d'onde, des cheveux traités avec la composition de l'invention et des cheveux non traités.
- La courbe correspondant aux cheveux traités doit montrer une réflectance dans la gamme des longueurs d'onde allant de 500 à 700 nanomètres supérieure à la courbe correspondant aux cheveux non traités.
- Cela signifie que, dans la gamme de longueur d'onde allant de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05 % de réflectance, et de préférence d'au moins 0,1%. Ceci n'empêche pas qu'il peut exister dans la gamme de longueur d'onde allant de 540 à 700 nanomètres, au moins une plage où la courbe de réflectance correspondant aux cheveux traités soit superposable, ou inférieure à la courbe de réflectance correspondant aux cheveux non traités.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

Au sens de la présente invention, et à moins qu'une indication différente ne soit donnée :

les radicaux « aryle » ou « hétéroaryle » peuvent être substitués par au moins un substituant porté par un atome de carbone, choisi parmi :

- un radical alkyle en $C_1$-$C_{16}$, de préférence en $C_1$-$C_6$ ;
- un atome d'halogène tel que chlore, fluor ou brome ;
- un groupement hydroxy ;
- un radical alcoxy en $C_1$-$C_2$ ;
- un radical alkylthio en $C_1$-$C_2$ ;
- un radical (poly)-hydroxyalcoxy en $C_2$-$C_4$ ;
- un radical amino ;
- un radical amino substitué par un ou deux radicaux alkyle, identiques ou différents, en $C_1$-$C_6$ éventuellement porteurs d'au moins :

  i) un groupement hydroxy,
  ii) un groupement amino

- un radical acylamino (-NR-COR') dans lequel le radical R est un atome d'hydrogène, un radical alkyle en $C_1$-$C_4$ éventuellement porteur d'au moins un groupement hydroxy et le radical R' est un radical alkyle en $C_1$-$C_2$ ;
- un radical carbamoyie ($(R)_2N$-CO-) dans lequel les radicaux R, identiques ou non, représentent un atome d'hydrogène, un radical alkyle en $C_1$-$C_4$ éventuellement porteur d'au moins un groupement hydroxy ;
- un radical alkylsulfonylamino ($R'SO_2$-NR-) dans lequel le radical R représente un atome d'hydrogène, un radical alkyle en $C_1$-$C_4$ éventuellement porteur d'au moins un groupement hydroxy et le radical R' représente un radical alkyle en $C_1$-$C_4$ ;
- un radical aminosulfonyle ($(R)_2N$-$SO_2$-) dans lequel les radicaux R, identiques ou non, représentent un atome d'hydrogène, un radical alkyle en $C_1$-$C_4$ éventuellement porteur d'au moins un groupement hydroxy,
- un radical carboxylique sous forme acide ou salifiée (de préférence avec un métal alcalin ou un ammonium, substitué ou non) ;
- un groupement cyano ;
- un groupement polyhalogénoalkyle comprenant de 1 à 6 atomes de carbones et de 1 à 6 atome d'halogène, identiques ou différents, le groupement polyhalogénoalkyle est par exemple le trifluorométhyle ;

  - la partie cyclique ou hétérocyclique d'un radical non aromatique peut être substituée par au moins un substituant porté par un atome de carbone choisi parmi les groupements :

- hydroxy,
- alkyle en $C_1$-$C_4$,
- alcoxy en $C_1$-$C_4$,
- (poly)hydroxyalcoxy en $C_2$-$C_4$,
- un radical alkylthio en $C_1$-$C_2$,
- RCO-NR'- dans lequel le radical R' est un atome d'hydrogène ou un radical alkyle en $C_1$-$C_4$ et le radical R est un atome d'hydrogène, un radical alkyle en $C_1$-$C_2$, ou amino substitué par deux groupements alkyle identiques ou différents en $C_1$-$C_4$ lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
- RCO-O- dans lequel le radical R est un radical alkyle en $C_1$-$C_4$, amino substitué par deux groupements alkyle identiques ou différents en $C_1$-$C_4$, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
- RO-CO- dans lequel le radical R est un radical alkyle en $C_1$-$C_4$, amino substitué par deux groupements alkyle identiques ou différents en $C_1$-$C_4$ lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;

  - un radical « aryle » représente un groupement mono ou polycyclique, condensé ou non, comprenant de 6 à 22 atomes de carbones, et dont au moins un cycle est aromatique ; préférentiellement le radical aryle est un phényle, biphényle, naphtyle, indényle, anthracényle, ou tétrahydronaphtyle ;

- un radical « diarylalkyle » représente un groupement comportant sur le même atome de carbone d'un groupement alkyle deux groupement aryle, identiques ou différents tel que diphénylméthyle ou 1,1-diphényléthyle ;
- un « radical hétéroaryle » représente un groupement mono ou polycyclique, condensé ou non, éventuellement cationique, comprenant de 5 à 22 chaînons, de 1 à 6 hétéroatomes choisis parmi l'atome d'azote, d'oxygène, de soufre et dont au moins un cycle est aromatique ; préférentiellement un radical hétéroaryle est choisis parmi acridinyle, benzimidazolyle, benzobistriazolyle, benzopyrazolyle, benzopyridazinyle, benzoquinolyle, benzothiazolyle, benzotriazolyle, benzoxazolyle, pyridinyle, tétrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle, xanthylyle et son sel d'ammonium ;
- un radical « dihétéroarylalkyle » représente un groupement comportant sur le même atome de carbone d'un groupement alkyle deux groupement hétéroaryle, identiques ou différents tel que difurylméthyle, 1,1-difuryléthyle, dipyrrolylméthyle, dithiénylméthyle ;
- un « radical cyclique » est un radical cycloalkyle non aromatique, mono ou polycyclique, condensé ou non, contenant de 5 à 22 atomes de carbone, pouvant comporter de 1 à plusieurs insaturations ; particulièrement le radical cyclique est un cyclohexyle ;
- un radical « cyclique stériquement encombré» est un radical cyclique, aromatique ou non, substitué ou non, encombré par effet ou contrainte stérique, comprenant de 6 à 14 chaînons, pouvant être pontés, à titre de radicaux stériquement encombrés on peut citer le bicyclo[1.1.0]butane, les mésytyles tels que le 1,3,5-triméthylpnényle, le 1,3,5-triterbutylphényle, le 1,3,5-isobutylphényle, le 1 ,3,5-trimétylsillylphényle et l'adamantyle ;
- un « radical hétérocyclique » est un radical non aromatique mono ou polycyclique, condensé ou non, contenant de 5 à 22 chaînons, comportant de 1 à 6 hétéroatomes choisis parmi l'atome d'azote, d'oxygène, de soufre et de sélénium ;
- un « radical alkyle » est un radical hydrocarboné en $C_1$-$C_{16}$, linéaire ou ramifié, de préférence en $C_1$-$C_8$ ;
- l'expression « éventuellement substitué » attribué au radical alkyle sous entend que ledit radical alkyle peut être substitué par un ou plusieurs radicaux choisis parmi les radicaux i) hydroxy, ii) alcoxy en $C_1$-$C_4$, iii) acylamino, iv) amino éventuellement substitué par un ou deux radicaux alkyle, identiques ou différents, en $C_1$-$C_4$, lesdits radicaux alkyle pouvant former avec l'atome d'azote qui les portent un hétérocycle comprenant de 5 à 7 chaînons, comprenant éventuellement un autre hétéroatome différent ou non de l'azote ;
- un « radical alcoxy » est un radical alkyle-oxy ou alkyl-O- pour lequel le radical alkyle est un radical hydrocarboné, linéaire ou ramifié, en $C_1$-$C_{16}$ préférentiellement en $C_1$-$C_8$ ;
- un « radical alkylthio » est un radical alkyl-S- pour lequel le radical alkyle est un radical hydrocarboné, linéaire ou ramifié, en $C_1$-$C_{16}$ préférentiellement en $C_1$-$C_8$ ;
- les bornes délimitant l'étendue d'une plage de valeurs sont comprises dans cette plage de valeurs ;
- un « sel d'acide organique ou minéral » est plus particulièrement choisi parmi un sel dérivé i) d'acide chlorhydrique HCl, ii) d'acide bromhydrique HBr, iii) d'acide sulfurique $H_2SO_4$, iv) d'acides alkylsulfoniques : Alk-$S(O)_2OH$ tels que d'acide méthylsulfonique et d'acide éthylsulfonique ; v) d'acides arylsulfoniques : Ar-$S(O)_2OH$ tel que d'acide benzène sulfonique et d'acide toluène sulfonique ; vi) d'acide citrique ; vii) d'acide succinique ; viii) d'acide tartrique ; ix) d'acide lactique, x) d'acides alcoxysulfiniques : Alk-O-S(O)OH tels que d'acide méthoxysulfinique et d'acide éthoxysulfinique ; xi) d'acides aryloxysulfiniques tels que d'acide toluèneoxysulfinique et d'acide phénoxysulfinique ; xii) d'acide phosphorique $H_3PO_4$; xiii) d'acide acétique $CH_3COOH$ ; xiv) d'acide triflique $CF_3SO_3H$ et xv) d'acide tétrafluoroborique $HBF_4$ ;
- un « contre-ion anionique » est un anion ou un groupement anionique associé à une charge cationique du colorant ; plus particulièrement le contre-ion anionique est choisi parmi i) les halogénures tels que le chlorure, le bromure ; ii) les nitrates ; iii) les sulfonates parmi lesquels les $C_1$-$C_6$ alkylsulfonates : Alk-$S(O)_2O^-$ tels que le méthylsulfonate ou mésylate et l'éthylsulfonate ; iv) les arylsulfonates : Ar-$S(O)_2O^-$ tel que le benzènesulfonate et le toluènesulfonate ou tosylate ; v) le citrate ; vi) le succinate ; vii) le tartrate ; viii) le lactate ; ix) les alkylsulfates : Alk-O-$S(O)O^-$ tels que le méthysulfate et l'éthylsulfate ; x) les arylsulfates : Ar-O-$S(O)O^-$ tels que le benzènesulfate et le toluènesulfate ; xi) les alcoxysulfates : Alk-O-$S(O)_2O^-$ tel que le méthoxy sulfate et l'éthoxysulfate ; xii) les aryloxysulfates : Ar-O-$S(O)_2O^-$, xiii) le phosphate ; xiv) l'acétate ; xv) le triflate ; et xvi) les borates tels que le tétrafluoroborate.

Les colorants fluorescents de formule (I) ou (II) sont des composés capables d'absorber dans le rayonnement UV ou visible à une longueur d'onde $\lambda_{abs}$ comprise entre 250 et 700 nm et capables de réémettrent dans le domaine du visible à une longueur d'onde d'émission $\lambda_{ém}$ comprise entre 400 et 700 nm.

De préférence les composés fluorescents de formule (I) ou (II) sont des colorants capables d'absorber dans le visible, $\lambda_{abs}$ comprise entre 400 et 700 nm, et de réémettre dans le visible, $\lambda_{ém}$ comprise entre 500 et 650 nm. Plus particulièrement les colorants fluorescents sont des colorants capables d'absorber à une longueur d'onde $\lambda_{abs}$ comprise entre 420 nm et 550 nm et de réémettre dans le visible à une longueur d'onde $\lambda_{ém}$ comprise entre 550 et 620 nm.

Un mode particulier concerne les colorants fluorescents de formule (I) ou (II) à fonction SY où Y représente un atome d'hydrogène, ou un métal alcalin. Avantageusement Y représente un atome d'hydrogène.

Les composés fluorescents de l'invention de formule (II) contiennent une fonction SY qui peut se trouver sous la forme covalente -S-Y ou ionique $-S^-\ Y^+$ selon la nature de Y et du pH du milieu.

Conformément à un autre mode de réalisation particulier de l'invention, dans la formule (II) précitée, Y est un groupement protecteur connu par l'homme du métier comme par exemple ceux décrits dans les ouvrages « Protective Groups in Organic Synthesis», T. W. Greene, John Willey & Sons ed., NY, 1981, pp.193-217 ; « Protecting Groups », P. Kocienski, Thieme, 3ème ed., 2005, chap. 5.

Particulièrement lorsque Y représente un groupement protecteur de la fonction thiol, Y est choisi parmi les radicaux suivants :

- ■ $(C_1-C_4)$alkylcarbonyle ;
- ■ $(C_1-C_4)$alkylthiocarbonyle ;
- ■ $(C_1-C_4)$alcoxycarbonyle ;
- ■ $(C_1-C_4)$alcoxythiocarbonyle ;
- ■ $(C_1-C_4)$alkylthio-thiocarbonyle ;
- ■ (di) $(C_1-C_4)$ (alkyl)aminocarbonyle ;
- ■ (di) $(C_1-C_4)$ (alkyl)aminothiocarbonyle;
- ■ arylcarbonyle comme phénylcarbonyle ;
- ■ aryloxycarbonyle ;
- ■ aryl$(C_1-C_4)$alkcoxycarbonyle ;
- ■ (di) $(C_1-C_4)$ (alkyl)aminocarbonyle comme diméthylaminocarbonyle ;
- ■ $(C_1-C_4)$(alkyl)arylaminocarbonyle ;
- ■ carboxy ;
- ■ $SO_3^-$ ; $M^+$ avec $M^+$ représentant un métal alcalin tel que le sodium ou le potassium, ou alors M' de la formule (II) et $M^+$ sont absents ;
- ■ aryle éventuellement substitué tel que le phényle, dibenzosubéryle, ou 1,3,5-cycloheptatriényle,
- ■ hétéroaryle éventuellement substitué ; dont notamment l'hétéroaryle cationiques ou non, comprenant de 1 à 4 hétéroatomes suivants :

  i) monocycliques à 5, 6 ou 7 chaînons tels que furanyle ou furyle, pyrrolyle ou pyrryle, thiophényle ou thiényle, pyrazolyle, oxazolyle, oxazolium, isoxazolyle, isoxazolium, thiazolyle, thiazolium, isothiazolyle, isothiazolium, 1,2,4-triazolyle, 1,2,4-triazolium, 1,2,3-triazolyle, 1,2,3-triazolium, 1,2,4-oxazolyle, 1,2,4-oxazolium, 1,2,4-thiadiazolyle, 1,2,4-thiadiazolium, pyrylium, thiopyridyle, pyridinium, pyrimidinyle, pyrimidinium, pyrazinyle, pyrazinium, pyridazinyle, pyridazinium, triazinyle, triazinium, tétrazinyle, tétrazinium, azépine, azépinium, oxazépinyle, oxazépinium, thiépinyle, thiépinium, imidazolyle, imidazolium ;

  ii) bicycliques à 8 à 11 chaînons tels que indolyle, indolinium, benzoimidazolyle, benzoimidazolium, benzoxazolyle, benzoxazolium, dihydrobenzoxazolinyle, benzothiazolyle, benzothiazolium, pyridoimidazolyle, pyridoimidazolium, thiénocycloheptadiényle, ces groupes mono ou bicycliques étant éventuellement substitués par un ou plusieurs groupements tels que $(C_1-C_4)$alkyle comme méthyle, ou polyhalogéno$(C_1-C_4)$alkyle comme trifluorométhyle ;

  iii) ou tricyclique ABC suivant :

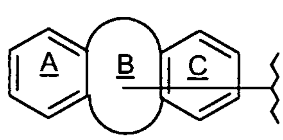

  dans lequel les deux cycles A, C comportent éventuellement un hétéroatome, et le cycle B est un cycle à 5, 6 ou 7 chaînons particulièrement à 6 chaînons et contient au moins un hétéroatome comme pypéridyle, pyranyle ;

- ■ hétérocycloalkyle éventuellement substitué, éventuellement cationique, le groupe hétérocycloalkyle représente notamment un groupe monocyclique saturé ou partiellement saturé à 5, 6 ou 7 chaînons comprenant de 1 à 4

hétéroatomes choisis parmi l'oxygène, le soufre et l'azote, tel que di/tétrahydrofuranyle, di/tétrahydrothiophényle, di/tétrahydropyrrolyle, di/tétrahydropyranyle, di/tétra/hexahydrothiopyranyle, dihydropyridyle, pipérazinyle, pipéridinyle, tétraméthylpipéridinyle, morpholinyle, di/tétra/hexahydroazépinyle, di/tétrahydropyrimidinyle ces groupes étant éventuellement substitués par un ou plusieurs groupes comme ($C_1$-$C_4$) alkyle, oxo ou thioxo ; ou l'hétérocycle représente le groupement suivant :

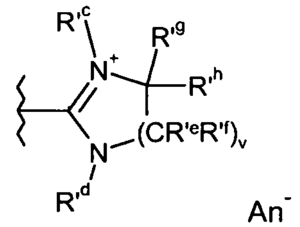

dans lequel $R'^c$, $R'^d$, $R'^e$, $R'^f$, $R'^g$ et $R'^h$, identiques ou différents représentent un atome d'hydrogène ou un groupement ($C_1$-$C_4$) alkyle, ou alors deux groupement $R'^g$ avec $R'^h$, et/ou $R'^e$ avec $R'^f$ forment un groupement oxo ou thioxo, ou alors $R'^g$ avec $R'^e$ forment ensemble un cycloalkyle ; et v représente un entier compris inclusivement entre 1 et 3 ; préférentiellement $R'^c$ à $R'^h$ représentent un atome d'hydrogène ; et $An^-$ représente un contre-ion ;

- ■ isothiouronium $-C(NR'^cR'^d)=N^+ R'^eR'^f$; $An^-$ avec $R'^c$, $R'^d$, $R'^e$ et $R'^f$, identiques ou différents représentent un atome d'hydrogène ou un groupement ($C_1$-$C_4$)alkyle ; préférentiellement $R'^c$ à $R'^f$ représentent un atome d'hydrogène ; et $An^-$ représente un contre-ion ;
- ■ isothiourée $-C(NR'^cR'^d)=NR'^e$ ; avec $R'^c$, $R'^d$ et $R'^e$, tels que définis précédemment ;
- ■ (di)aryl($C_1$-$C_4$)alkyle éventuellement substitué tel que le 9-anthracénylméthyle, phénylméthyle ou diphénylméthyle éventuellement substitué par un plusieurs groupements notamment choisis parmi ($C_1$-$C_4$) alkyle, ($C_1$-$C_4$) alcoxy comme le méthoxy, hydroxy, ($C_1$-$C_4$) alkylcarbonyle, ou (di) ($C_1$-$C_4$) (alkyl)amino comme le diméthylamino ;
- ■ (di)hétéroaryl($C_1$-$C_4$)akyle éventuellement substitué, le groupe hétéroaryle est notamment, cationique ou non, monocyclique, comprenant 5 ou 6 chaînons et de 1 à 4 hétéroatomes choisis parmi l'azote, l'oxygène et le soufre, tels que les groupes pyrrolyle, furanyle, thiophényle, pyridyle, pyridyle N-oxyde tels que le 4-pyridyle ou 2-pyridyl-N-oxyde, pyrylium, pyridinium, triazinyle, éventuellement substitué par un ou plusieurs groupement tel que alkyle particulièrement méthyle, avantageusement le (di)hétéroaryl($C_1$-$C_4$)akyle est (di)hétéroarylméthyle ou (di)hétéroaryléthyle ;
- ■ $CR^1R^2R^3$ avec $R^1$, $R^2$ et $R^3$ identiques ou différents, représentant un atome d'halogène ou un groupe choisi parmi :
  - ($C_1$-$C_4$) alkyle ;
  - ($C_1$-$C_4$) alcoxy ;
  - aryle éventuellement substitué tel que phényle éventuellement substitué par un ou plusieurs groupements comme ($C_1$-$C_4$)alkyle, ($C_1$-$C_4$)alcoxy, hydroxy ;
  - hétéroaryle éventuellement substitué tel que thiophényle, furanyle, pyrrolyle, pyranyle, pyridyle, éventuellement substitué par un groupement ($C_1$-$C_4$)alkyle ;
  - $P(Z^1)R'^1R'^2R'^3$ avec $R'^1$, et $R'^2$ identiques ou différents représentent un groupement hydroxy, ($C_1$-$C_4$)alcoxy ou alkyle, $R'^3$ représente un groupement hydroxy ou ($C_1$-$C_4$)alcoxy, et $Z^1$ représente un atome d'oxygène ou de soufre ;
- ■ cyclique stériquement encombré tel que le groupe adamantyle ; et
- ■ alcoxy($C_1$-$C_4$)alkyle éventuellement substitué tels que le méthoxyméthyle (MOM), éthoxyéthyle (EOM) et l'isobutoxyméthyle.

Selon un mode de réalisation particulier les colorants fluorescents thiols protégés de formule (II) comportent un groupement Y i) hétéroaryle monocyclique à 5 ou 6 chaînons aromatiques, cationiques comprenant de 1 à 4 hétéroatomes choisis parmi l'oxygène, le soufre et l'azote, tels que oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinyle, pyrazinium, pyridazinium, triazinium, tétrazinium, oxazépinium, thiépinyle, thiépinium, imidazolium ; ii) hétéroaryle bicyclique à 8 à 11 chaînons cationique tels que indolinium, benzoimidazolium, benzoxazolium, benzothiazolium, ces groupes hétéroaryle mono ou bicycliques étant éventuellement substitués par un ou plusieurs groupements tels que alkyle comme

méthyle, ou polyhalogéno($C_1$-$C_4$)alkyle comme trifluorométhyle ; iii) ou hétérocyclique suivant :

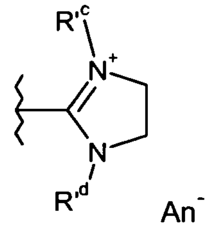

dans lequel R'c et R'd, identiques ou différents, représentent un atome d'hydrogène ou un groupement ($C_1$-$C_4$)alkyle ; préférentiellement R'c à R'd représentent un groupement ($C_1$-$C_4$)alkyle tel que méthyle ; et $An^-$ représente un contre-ion.

Particulièrement Y représente un groupement choisi parmi oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinium, pyridazinium, triazinium et imidazolium, benzoimidazolium, benzoxazolium, benzothiazolium, ces groupements étant éventuellement substitués par un ou plusieurs groupes ($C_1$-$C_4$) alkyle notamment méthyle.

En particulier les colorant de formule (II) possèdent un groupe Y qui représente un métal alcalin ou un groupement protecteur tel que:

- ($C_1$-$C_4$)alkylcarbonyle comme méthylcarbonyle ou éthylcarbonyle ;
- arylcarbonyle comme phénylcarbonyle ;
- ($C_1$-$C_4$)alcoxycarbonyle ;
- aryloxycarbonyle ;
- aryl($C_1$-$C_4$)alcoxycarbonyle ;
- (di) ($C_1$-$C_4$) (alkyl)aminocarbonyle comme diméthylaminocarbonyle ;
- ($C_1$-$C_4$)(alkyl)arylaminocarbonyle ;
- aryle éventuellement substitué tel que le phényle ;
- hétéroaryle monocyclique cationique à 5, 6 chaînons tels que pyrylium, pyridinium, pyrimidinium, pyrazinium, pyridazinium, triazinium, imidazolium ; ces groupements étant éventuellement substitué par un ou plusieurs, identiques ou différents, groupes ($C_1$-$C_4$)alkyle tel que méthyle ;
- hétéroaryle bicyclique cationique à 8 à 11 chaînons tels que benzoimidazolium, ou le benzoxazolium ; ces groupements étant éventuellement substitué par un ou plusieurs, identiques ou différents, groupes ($C_1$-$C_4$)alkyle tel que méthyle ;
- hétérocycle cationique de formule suivante :

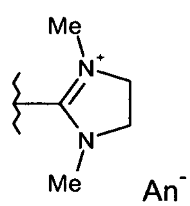

- isothiouronium $-C(NH_2)=N^+H_2$, $An^-$ ;
- isothiourée $-C(NH_2)=NH$ ;
- $SO_3$ , $M^+$ avec $M^+$ avec $M^+$ représentant un métal alcalin tel que le sodium ou le potassium, ou alors M' de la formule (II) et $M^+$ sont absents.

Selon un mode particulier de l'invention, les colorants fluorescents de l'invention sont de formule (Ia) ou (IIa), possédant chacun un groupement éthylène qui relie la partie pyridinium au phényle en position ortho ou para soit en positions 4-4', 4-2' ou 2-4' :

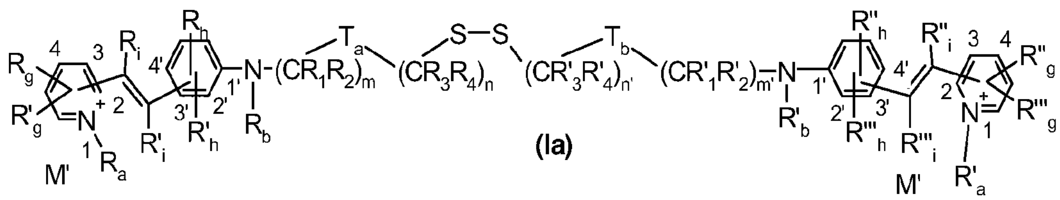

(Ia)

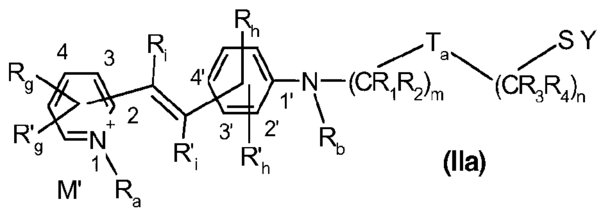

(IIa)

formule (Ia) et (IIa) avec $R_1$, $R_2$, $R_3$, $R_4$, $R_a$, $R_b$, $R_i$, $R'_i$, $R_g$, $R'_g$, $R_h$, $R'_h$, m, n, Y, $R'_1$, $R'_2$, $R'_3$, $R'_4$, $R'_a$, $R'_b$, $R''_i$, $R'''_i$, $R''_g$, $R'''_g$, $R''_h$, $R'''_h$, m' et n' sont tels que définis précédemment.

Un autre mode de réalisation particulier de l'invention concerne des colorants disulfure de formule (I) ou (Ia) symétriques ie $R_a$, $R_b$, $R_g$, $R'_g$, $R_h$, $R'_h$, $R_i$, $R'_i$, $R_i$, $R_2$, $R_3$. $R_4$, $T_a$, m, n sont identiques à $R'_a$, $R'_b$, $R''_g$, $R'''_g$, $R''_h$, $R'''_h$, $R''_i$, $R''_i$, $R'_1$, $R'_2$, $R'_3$, $R'_4$, $T_b$, m' et n' respectivement.

A titre d'exemple de colorants fluorescents thiols, on peut citer notamment les colorants suivants :

| | |
|---|---|
| 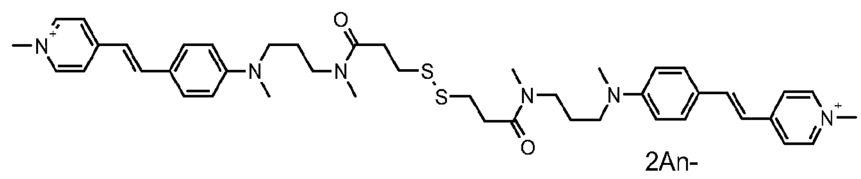 | |
| **1** | |
| 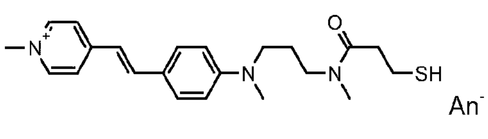 | 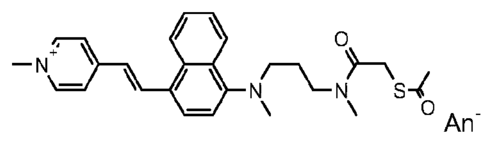 |
| **2** | **3** |
| 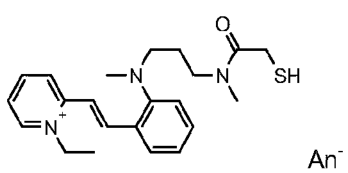 | 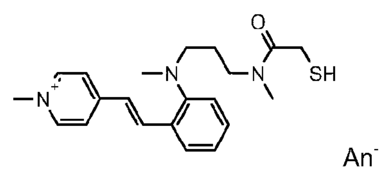 |
| **3** | **4** |
| 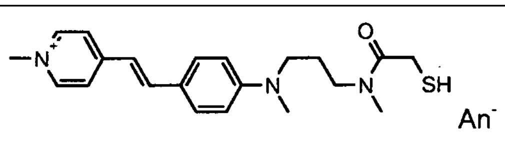 | 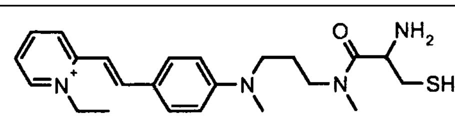 |
| **5** | **6** |

(suite)

<table>
<tr><td>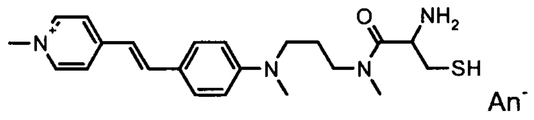</td><td>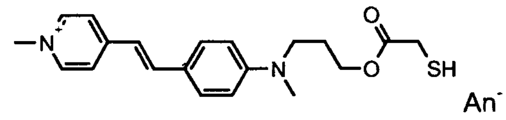</td></tr>
<tr><td>7</td><td>8</td></tr>
<tr><td>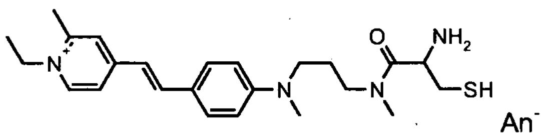</td><td>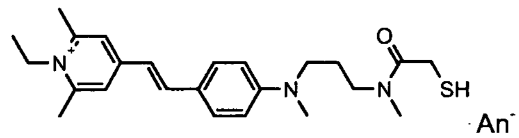</td></tr>
<tr><td>9</td><td>10</td></tr>
<tr><td>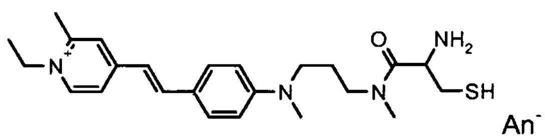</td><td>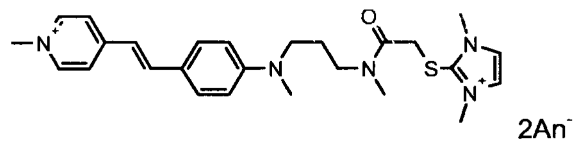</td></tr>
<tr><td>11</td><td>12</td></tr>
<tr><td colspan="2">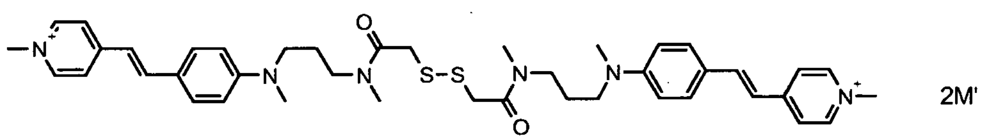</td></tr>
<tr><td colspan="2">13</td></tr>
<tr><td colspan="2">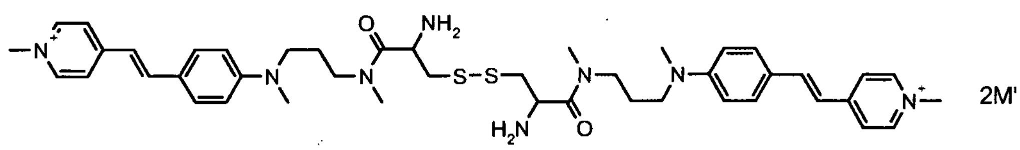</td></tr>
<tr><td colspan="2">14</td></tr>
<tr><td colspan="2">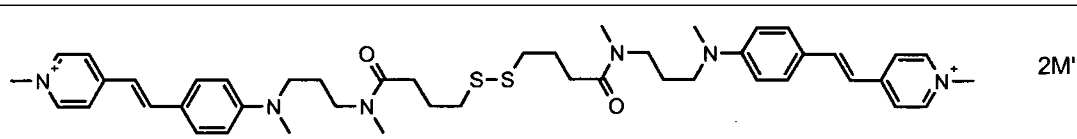</td></tr>
<tr><td colspan="2">15</td></tr>
<tr><td colspan="2">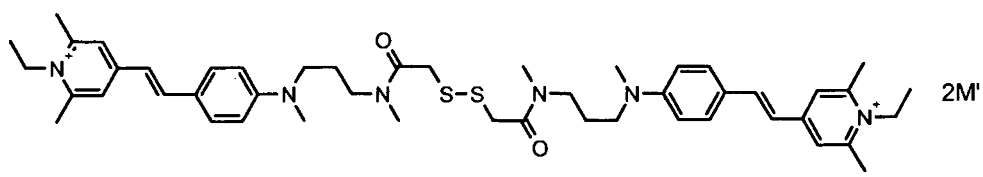</td></tr>
<tr><td colspan="2">16</td></tr>
<tr><td colspan="2">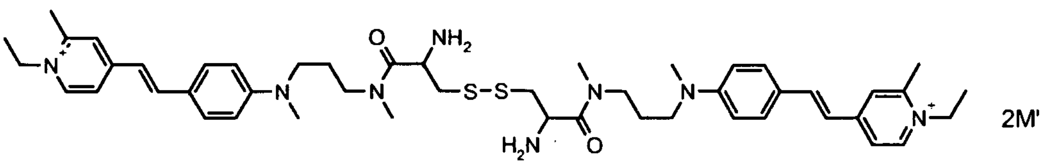</td></tr>
<tr><td colspan="2">17</td></tr>
</table>

(suite)

| |
|---|
| 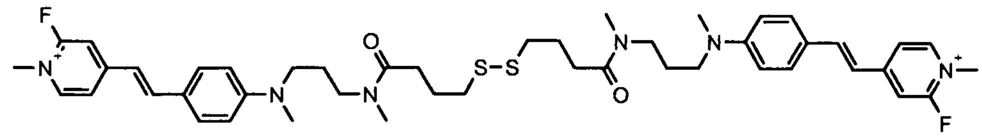 2M' |
| **18** |
| 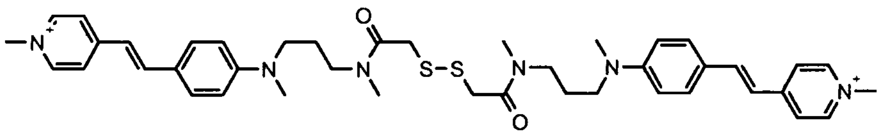 2M' |
| **19** |
| 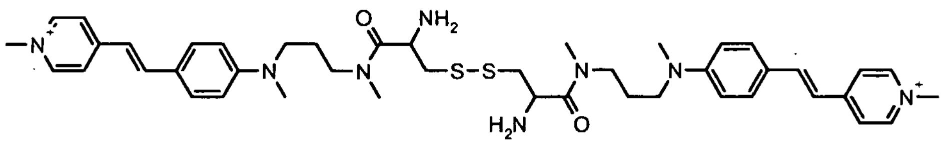 2M' |
| **20** |
| 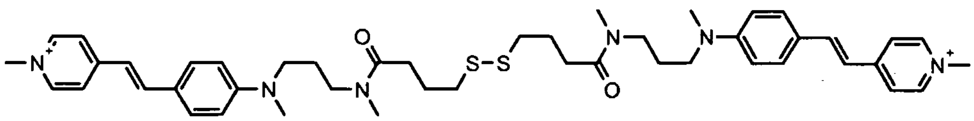 2M' |
| **21** |
| 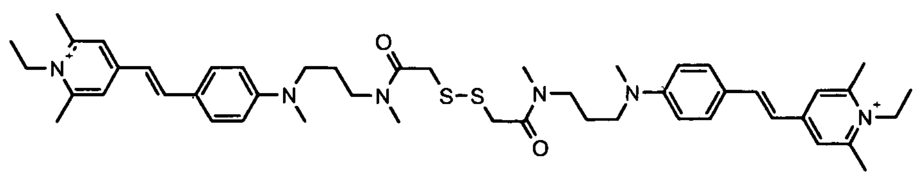 2M' |
| **22** |
| 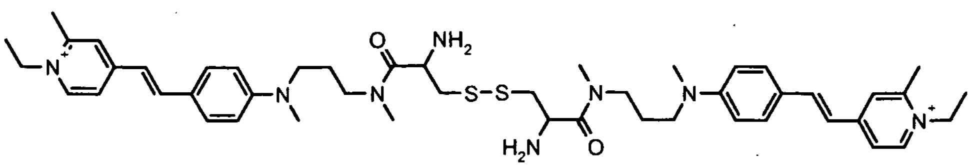 2M' |
| **23** |
| 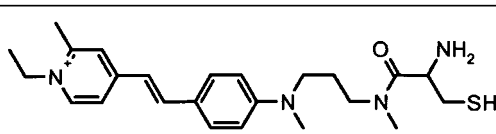 An⁻ |
| **24** |
| 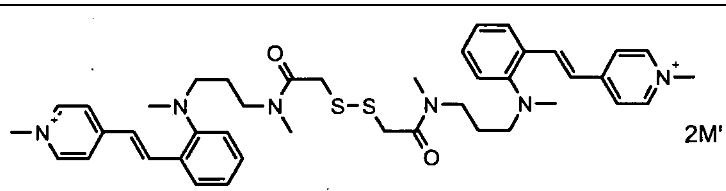 2M' |

(suite)

| 25 |
|---|
| 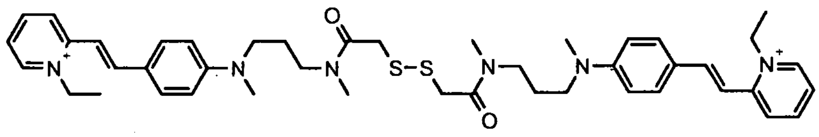 2M' |
| 26 |
| 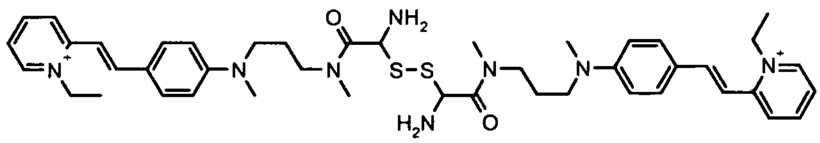 2M' |
| 27 |
| 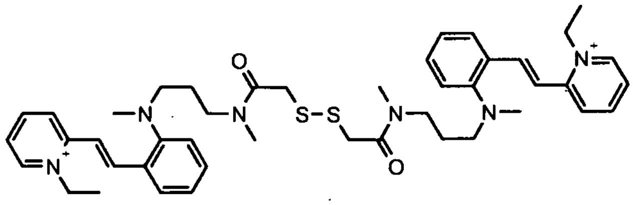 2M' |
| 28 |

avec M', An⁻, identiques ou différents, représentant un contre-ion anionique.

Avec M' et $An^-$, identiques ou différents, représentent notamment un contre-ion tel que les halogénures, tels que le chlorure, le bromure ; les nitrates ; les sulfonates parmi lesquels les $C_1$-$C_6$ alkylsulfonates : Alk-S(O)$_2$O$^-$ tels que le méthylsulfonate ou mésylate et l'éthylsulfonate ; les arylsulfonates : Ar-S(O)$_2$O$^-$ tel que le benzènesulfonate et le toluènesulfonate ou tosylate ; le citrate ; le succinate ; le tartrate ; le lactate ; les alkylsulfates : Alk-O-S(O)O$^-$ tels que le méthylsulfate et l'éthylsulfate ; les arylsulfates : Ar-O-S(O)O$^-$ tels que le benzènesulfate et le toluènesulfate ; les alcoxysulfates : Alk-O-S(O)$_2$O$^-$ tel que le méthoxy sulfate et l'éthoxysulfate ; les aryloxysulfates : Ar-O-S(O)$_2$O$^-$, le phosphate ; l'acétate ; le triflate ; et les borates tels que le tétrafluoroborate.

Les colorants thiols protégés de formule **(II)** peuvent être synthétisés en deux étapes. La première étape consistant à préparer le colorant thiol non protégé selon les méthodes connues de l'homme de l'art comme par exemple « Thiols and organic Sulfides », « Thiocyanates and Isothiocyanates, organic », Ullmann's Encyclopedia, Wiley-VCH, Weinheim, 2005. Et la deuxième étape consistant à protéger la fonction thiol selon les méthodes classiques connues par l'homme du métier pour conduire aux colorants thiols protégés de formule **(II)**. A titre d'exemple pour protéger la fonction thiol -SH du colorant thiol on peut utiliser les méthodes des ouvrages « Protective Groups in Organic Synthesis », T. W. Greene, John Willey & Sons ed., NY, 1981, pp. 193-217 ; « Protecting Groups », P. Kocienski, Thieme, 3ème ed., 2005, chap. 5.

Nous pouvons illustrer cette méthode par la méthode consistant i) à générer des colorants fluorescents thiols par réduction d'un colorant fluorescent à deux chromophores portant une fonction disulfure -S-S- tels que **(I)** et ii) à protéger selon les méthodes classiques ladite fonction thiol les composés **(II')** pour accéder aux colorants fluorescents thiols protégés de formule **(II")** :

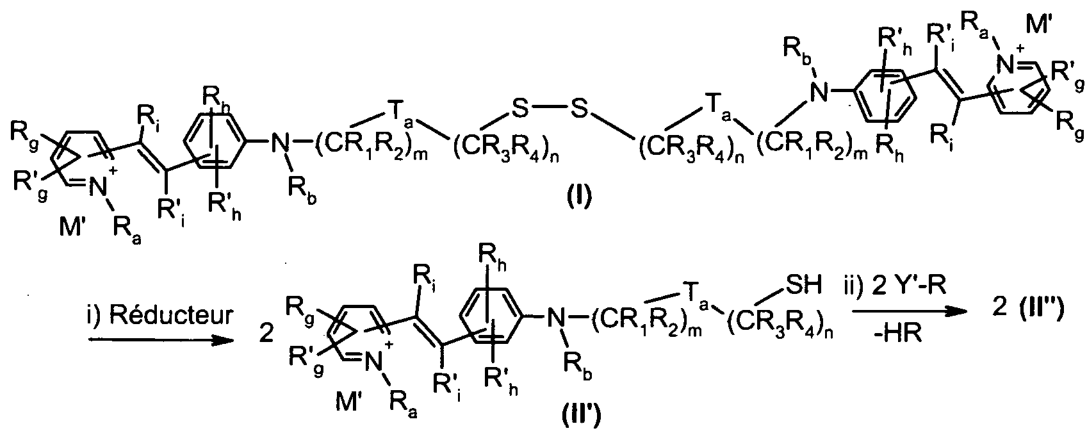


avec $R_1$, $R_2$, $R_3$, $R_4$, $R_a$, $R_b$, $R_g$, $R'_g$, $R_h$, $R'_h$, $R_i$, $R'_i$, $T_a$, m, n et M' sont tels que définis précédemment et R représente un groupe partant nucléofuge, comme par exemple mésylate, tosylate, triflate ou halogénure, et Y' représente un groupe protecteur de la fonction thiol.

Selon une autre possibilité, on peut faire réagir un composé thiol protégé **(b)** par un groupement protecteur Y' tel que défini précédemment préparé selon une des procédures décrites dans les ouvrages cités précédemment, ledit composé thiol protégé comprenant au moins une fonction nucléophile avec une quantité suffisante, préférentiellement équimolaire, d'un "chromophore fluorescent réactif" ou d'un composé comprenant un tel "chromophore fluorescent réactif" **(a)**. En d'autres termes **(a)** comprend une fonction nucléofuge pour former une liaison covalente $\Sigma$ comme cela peut être schématisé ci-dessous:

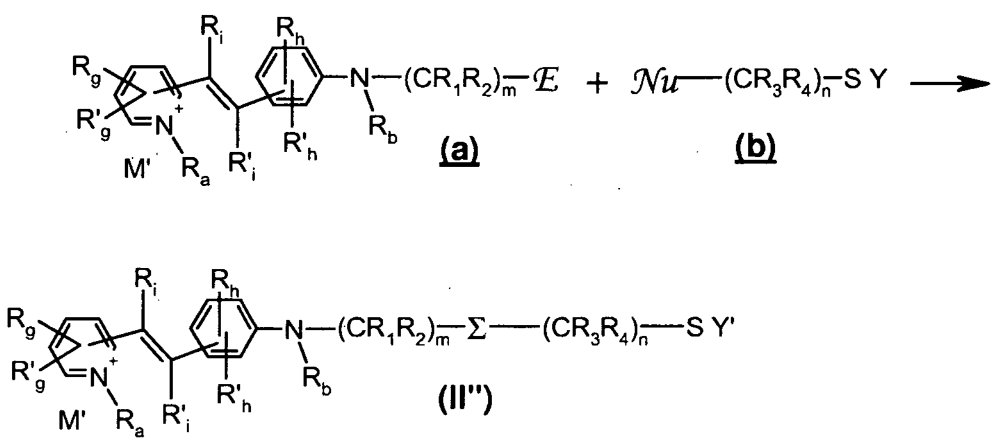


avec $R_1$, $R_2$, $R_3$, $R_4$, $R_a$, $R_b$, $R_g$, $R'_g$, $R_h$, $R'_h$, $R_i$, $R'_i$, Y', m, n et M' sont tels que définis précédemment ; Nu représentant un groupement nucléophile aminé, oxygéné tel que amine primaire, hydroxy, thiol ; E représentant un groupement électrophile comprenant un carbonyle, un thiocarbonyle tel qu'un halogénure d'acyle, thiohalogénure d'acyle, un ester, un thioester. Il suit que $\Sigma$ représente un enchaînement de radicaux choisis parmi $-SO_2-$, -O-, -S-, -N(R)-, -CO-, avec R, représente un atome d'hydrogène, un radical alkyle en $C_1$-$C_4$, hydroxyalkyle en $C_1$-$C_4$; ou un aryl($C_1$-$C_4$)alkyle, notamment $\Sigma$ représente un groupement choisi parmi -C(O)-N(R)-, -C(O)-O-, -C(S)-O-, -C(O)-S-, -C(S)-S- ou -C(S)-N(R)-.

On pourra également utiliser un réactif thiol Y-SH comprenant un groupement Y dont la fonction nucléophile SH peut réagir sur l'atome de carbone en alpha de l'atome d'halogène porté par le chromophore fluorescent **(a')** pour conduire au colorant fluorescent thiol protégé de formule **(II):**

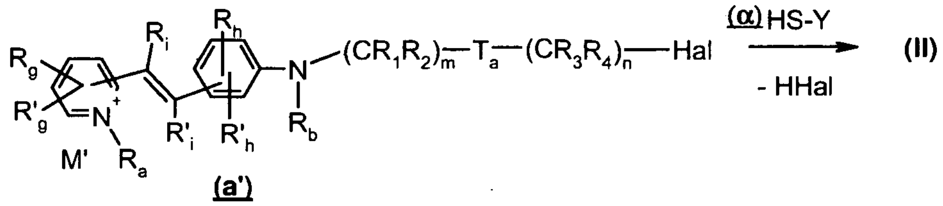


avec $R_1$, $R_2$, $R_3$, $R_4$, $R_a$, $R_b$, $R_g$, $R'_g$, $R_h$, $R'_h$, $R_i$, $R'_i$, $T_a$, Y, m, n et M' sont tels que définis précédemment, et Hal représentant un atome d'halogène nucléofuge tel que le brome, l'iode ou le chlore.

On pourra également utiliser un thioacide (α) (par exemple l'acide de thioacétique) qui agira sur halogénure porté par le chromophore fluorescent **(a')** :

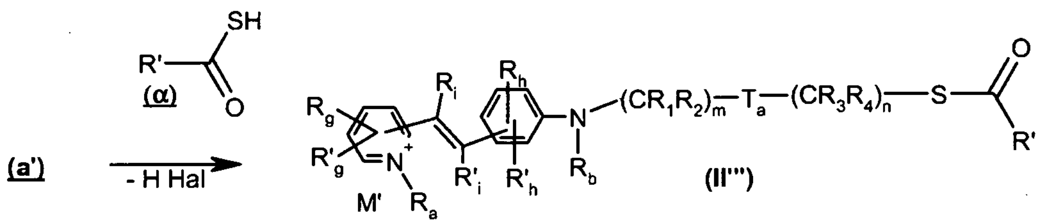


avec $R_1$, $R_2$, $R_3$, $R_4$, $R_a$, $R_b$, $R_g$, $R'_g$, $R_h$, $R'_h$, $R_i$, $R'_i$, m, n, $T_a$, M' sont tels que définis précédemment et R' représentant un groupement $(C_1\text{-}C_6)$alkyle.

Plus particulièrement on pourra substituer un groupement partant nucléofuge par un groupement thiourée (S=C(NRR)NRR) pour générer les isothiouroniums. Par exemple si le groupement thiourée est une thioimidazolinium (β), le schéma réactionnel est le suivant :

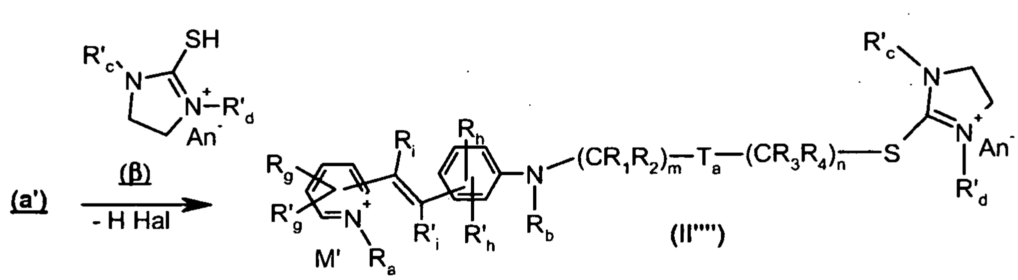


avec $R_1$, $R_2$, $R_3$, $R_4$, $R_a$, $R_b$, $R'_c$, $R'_d$, $R_g$, $R'_g$, $R_h$, $R'_h$, $R_i$, $R'_i$, m, n, $T_a$, $An^-$ et M' sont tels que définis précédemment.

Une variante est d'utiliser à la place de l'halogénure comprenant le chromophore fluorescent **(a')** un chromophore comprenant un autre type de nucléofuge tel que le tosylate, mésylate.

Selon une autre variante il est possible de générer un intermédiaire imidazoline à partir de l'halogénure comprenant le chromophore fluorescent **(a')** et une thioimidazoline **(b')** pour conduire après alkylation par un réactif R-Gp avec R représentant un groupement alkyle et Gp un groupe partant tel qu'un halogène comme le chlore, brome, iode, ou un groupement mésylate ou tosylate, pour conduire au colorant de formule **(II'''')**.

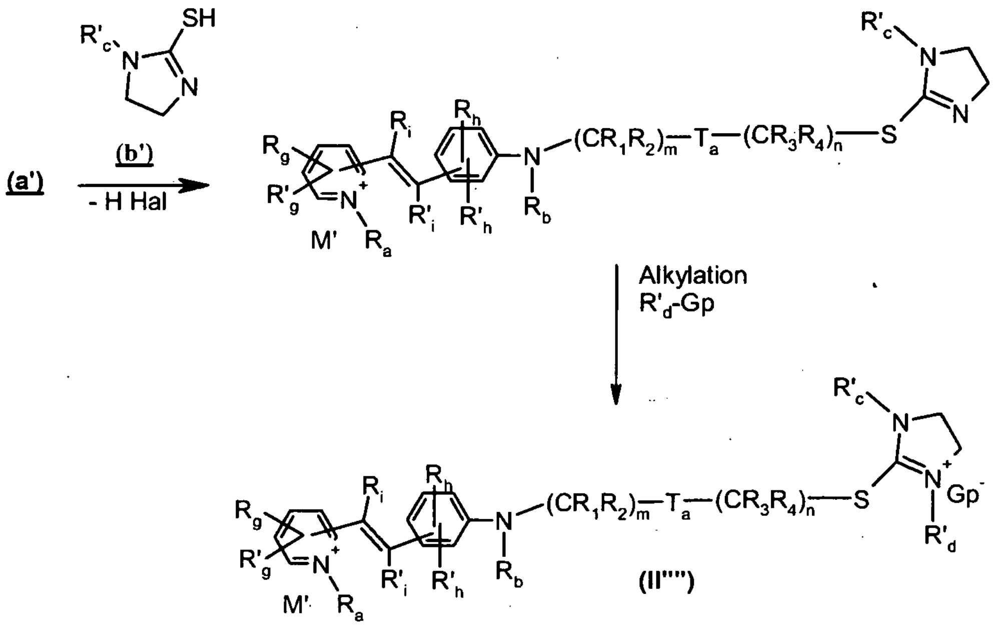


On peut également substituer un groupement partant nucléofuge par les sels de thiosulfate (exemple thiosulfate de sodium ou potassium) pour générer les sels de Bunte ($-SSO^{-}_{s}$, $Na^+$ ou $K^+$) :

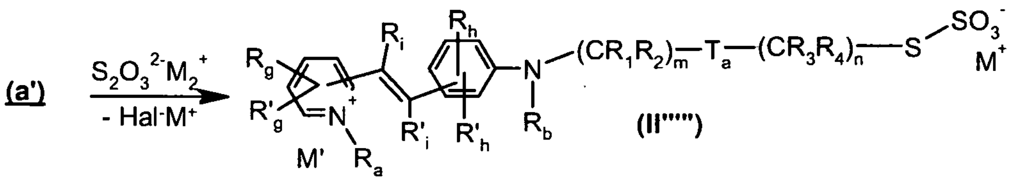


avec $R_1$, $R_2$, $R_3$, $R_4$, $R_a$, $R_b$, $R_g$, $R'_g$, $R_h$, $R'_h$, $R_i$, $R'_i$, m, n, $T_a$, M' sont tels que définis précédemment et $M^+$ représentant un métal alcalin tel que le sodium ou potassium.

Conformément à une autre possibilité, certains colorants fluorescents thiols protégés **(II")** peuvent être obtenus en faisant réagir un composé thiol protégé, avec un composé portant une fonction nucléofuge ou carbonyle de type halogénure d'acyle (**d**) mis à réagir avec un chromophore fluorescent porteur d'une fonction nucléophile (**c**). de type amine primaire ou secondaire :

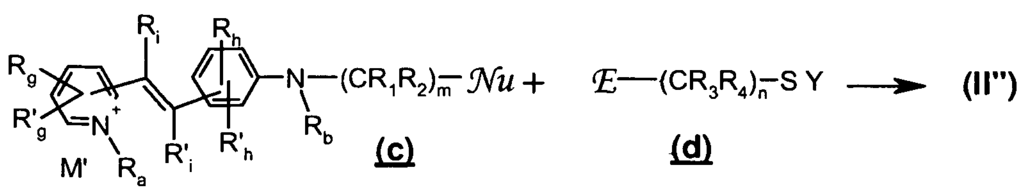


avec $R_1$, $R_2$, $R_3$, $R_4$, $R_a$, $R_b$, $R_g$, $R'_g$, $R_h$, $R'_h$, $R_i$, $R'_i$, $T_a$, Y, m, n, M', $\mathcal{Nu}$ et **(II")** tels que définis précédemment et $\mathcal{E}$ représentant un groupement électrophile comprenant un carbonyle, un thiocarbonyle tel qu'un halogénure d'acyle, thiohalogénure d'acyle, un ester, un thioester. Il suit que Σ représente un enchaînement de radicaux choisis parmi $-SO_2-$, -O-, -S-, -N(R)-, -CO-, avec R, représent un atome d'hydrogène, un radical alkyle en $C_1$-$C_4$, hydroxyalkyle en $C_1$-$C_4$; ou un aryl($C_1$-$C_4$)alkyle, notamment Σ représente un groupement choisi parmi -N(R)-C(O)-, -O-C(O)-, -O-C(S)-, -S-C (O)-, S-C(S)- ou -N(R)-C(S)-.

Une variante est d'utiliser une thiolactone pouvant subir l'attaque nucléophile d'un groupe amino porté par le chromophore styrylpyridinium (c') pour conduire au colorant fluorescent thiol **(II'$_a$)** qui peut soit être protégé, soit être

métallé pour donner respectivement les colorants **(II"$_a$)** et **(II"$_a$met)** comme l'illustre le schéma suivant:

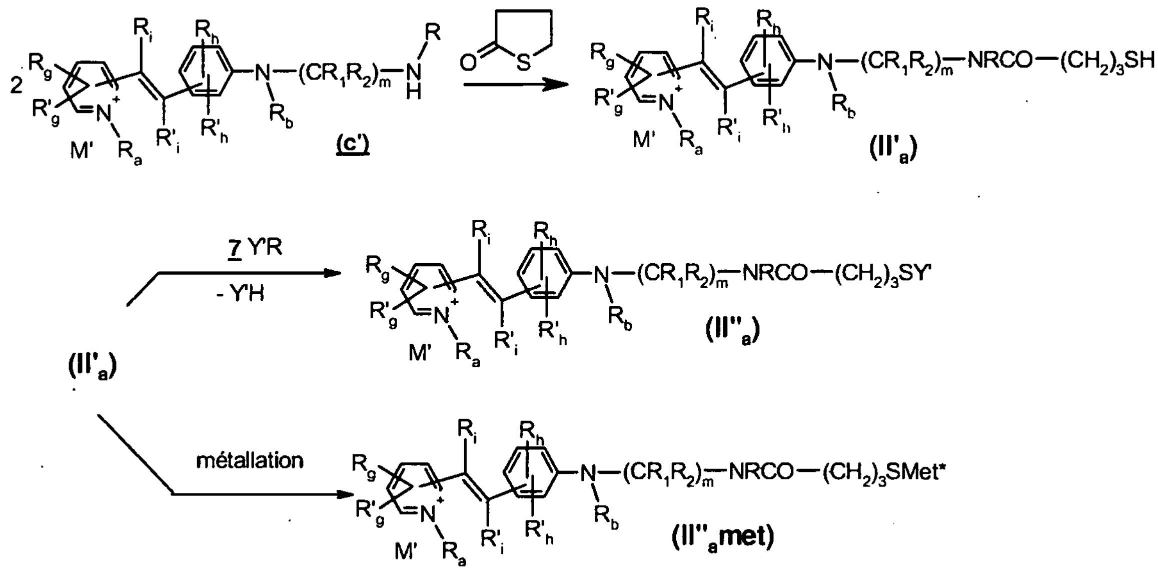


Conformément à une autre possibilité, les colorants fluorescents thiols protégés de formule **(II)** peuvent être obtenus par réaction d'un composé comprenant un groupement thiol protégé par un groupement Y, et un groupement hydroxy activé préalablement en groupement partant nucléofuge **(d')**, comme par exemple mésylate, tosylate, triflate ou halogénure avec un chromophore fluorescent **(c')** portant une fonction nucléophile amine primaire lorsque $R_b$ représente un atome d'hydrogène, ou amine secondaire.

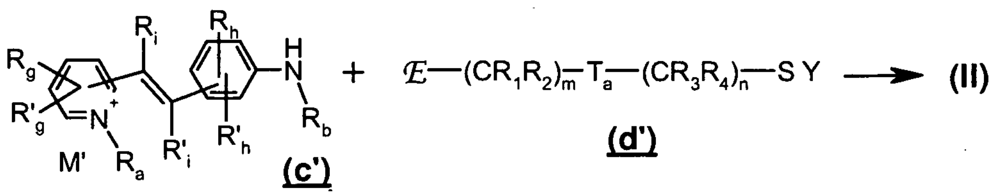


Avec $R_1$, $R_2$, $R_3$, $R_4$, $R_a$, $R_b$, $R_g$, $R'_g$, $R_h$, $R'_h$, $R_i$, $R'_i$, $T_a$, Y, m, n, et M' sont tels que définis précédemment et

$\mathcal{E}$ représente un groupement nucléofuge comme par exemple mésylate, tosylate, triflate ou halogénure.

Conformément à une autre possibilité, les colorants fluorescents thiols de formule **(II)** selon l'invention peuvent être obtenus par réaction d'un composé comprenant un groupement thiol protégé et un groupement électrophile **(f)** avec un composé comprenant un groupement nucléophile. A titre d'exemple, on pourra condenser un aldéhyde ou cétone lorsque G représente un atome d'oxygène avec un « méthylène activé » tels que l'alkylpyridinium **(e)** pour générer un liaison éthylène >C=C<. Cette réaction est communément appelée condensation de « Knoevenagel ». Par « méthylènes activés » on sous entend ceux par exemple mentionnés dans le brevet DE19951134 on peut citer particulièrement le 1,2-dialkylpyridinium et notamment le 1,2-diméthylpyridinium :

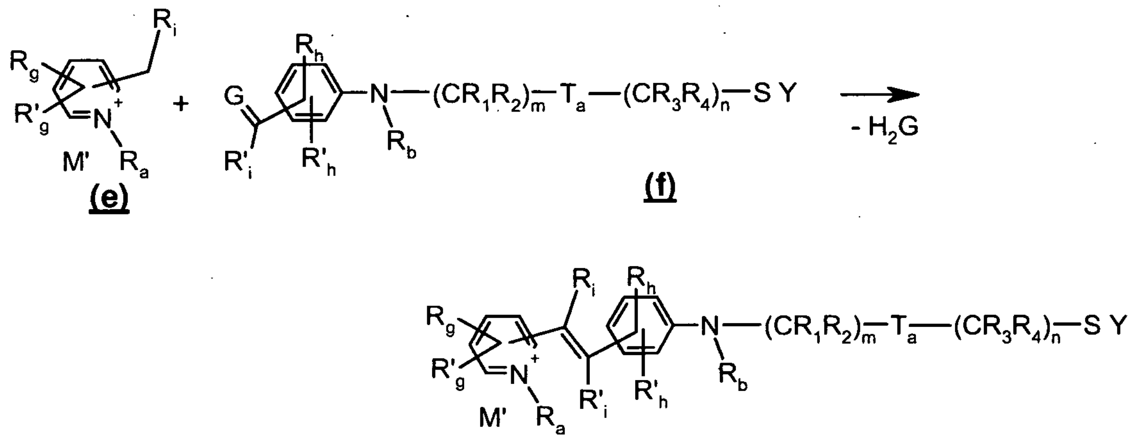


(e) (f)

avec $R_1$, $R_2$, $R_3$, $R_4$, $R_a$, $R_b$, $R_g$, $R'_g$, $R_i$, $R'_h$, $R'_i$, $T_a$, Y, m, n, M' tels que définis précédemment et G représentant un atome d'oxygène, de soufre ou un groupement NR" avec R" représentant un atome d'hydrogène ou un groupement alkyle.

Une autre variante à ce schéma réactionnel est de réaliser la réaction de couplage de « Knoevenagel » ultérieurement ie à partir de l'intermédiaire **(f')**, le groupement amino R'NH peut réagir avec un équivalent de réactif $\mathcal{E}$ -$(CR_3R_4)$- $\mathcal{E}$ pour conduire à **(i')** qui lui-même peut réagir avec un dérivé thiol protégé Y'SH pour donner l'intermédiaire aldéhyde **(i')**. Cet intermédiaire peut subir la réaction de condensation de « Knoevenagel » avec l'alkylpyridinium **(e)** pour conduire au colorant fluorescent thiol protégé **(II'')**.

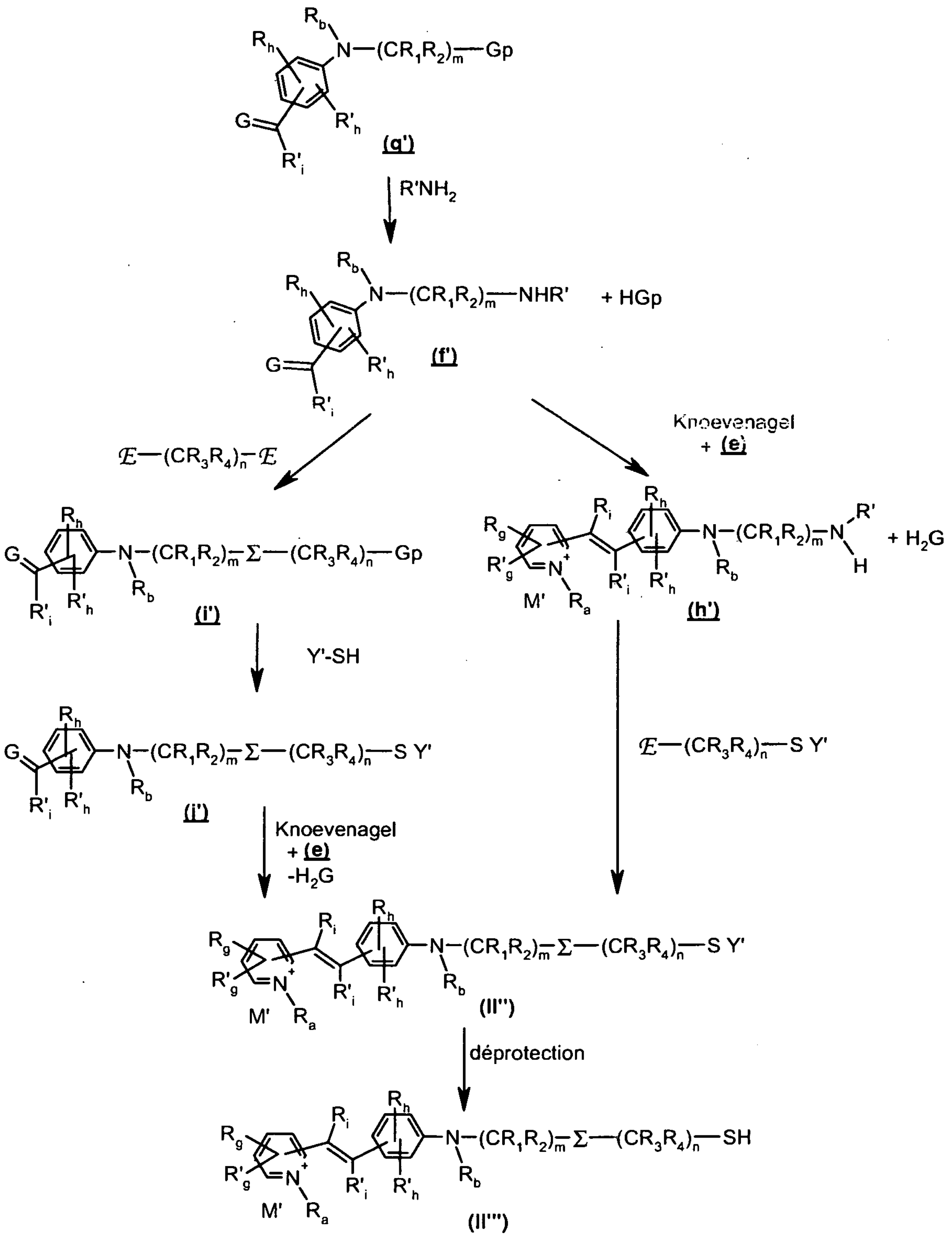


avec R', $R_1$, $R_2$, $R_3$, $R_4$, $R_a$, $R_b$, $R_g$, $R'_g$, $R_i$, $R'_h$, $R_i$, $R'_i$, $\mathcal{E}$ , m, n, Y', et M' tels que définis, et Σ représente notamment un N(R)-C(O)-, ou -N(R)-C(S)-.

On pourra se référer à l'ouvrage Advanced Organic Chemistry, « Reactions, Mechanisms and Structures », J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Les colorants fluorescents thiols formés peuvent être transformés en colorants fluorescents thiols protégés -S Y' par la protection du thiol -SH en utilisant les groupements protecteurs classiques. Les colorants fluorescents thiols peuvent également êtres métallés en utilisant les méthodes classiques connues par l'homme du métier telles que celles décrites dans Advanced Organic Chemistry, « Reactions, Mechanisms and Structures », J. March, 4ème Ed, John Willey

& Sons, NY, 1992.

Les colorants thiols protégés peuvent êtres déprotégés par voies classiques telles que celles décrites dans les ouvrages « Protective Groups in Organic Synthesis », T. W. Greene, John Willey & Sons ed., NY, 1981 ; « Protecting Groups », P. Kocienski, Thieme, 3ème ed., 2005.

Les réactifs de départs sont commerciaux ou accessibles par voies classiques connues par l'homme du métier. A titre d'exemple pour synthétiser le colorant fluorescent à deux chromophores portant une fonction disulfure -S-S- (I'), on peut partir d'un réactif difonctionnalisé **(k),** comprenant an position 1 un groupement nucléofuge G-R avec R représentant un groupement $(C_1-C_4)$alkyle, mésylate, tosylate, ou triflate et G représentant un atome d'oxygène ou de soufre ou un halogène tel que le fluor, le chlore, l'iode, et comprenant en position 2 ou 4 un groupement électrophile aldéhyde ou thioaldéyde. Deux équivalents de ce réactif peuvent réagir avec la diamine-dialkyl-disulfure **(l)** pour conduire après condensation au dicétone/thioalcétone disulfure **(m)** pouvant se condenser avec deux équivalents d'alkylpyridinium **(e)** pour former le composé **(I').**

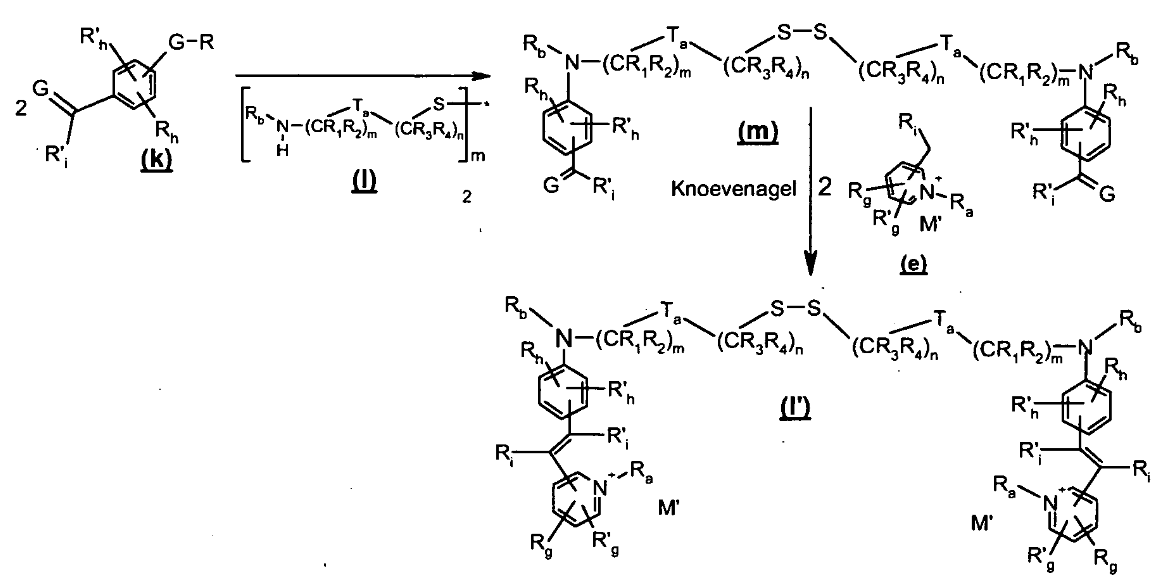


On pourra se référer à l'ouvrage Advanced Organic Chemistry, J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Les colorants disulfures disymétriques de formule **(I)** peuvent être synthétisés en une étape en faisant réagir un colorant fluorescent thiol non protégé avec un colorant fluorescent thiol protégé pour former le colorant disulfure de formule **(I).**

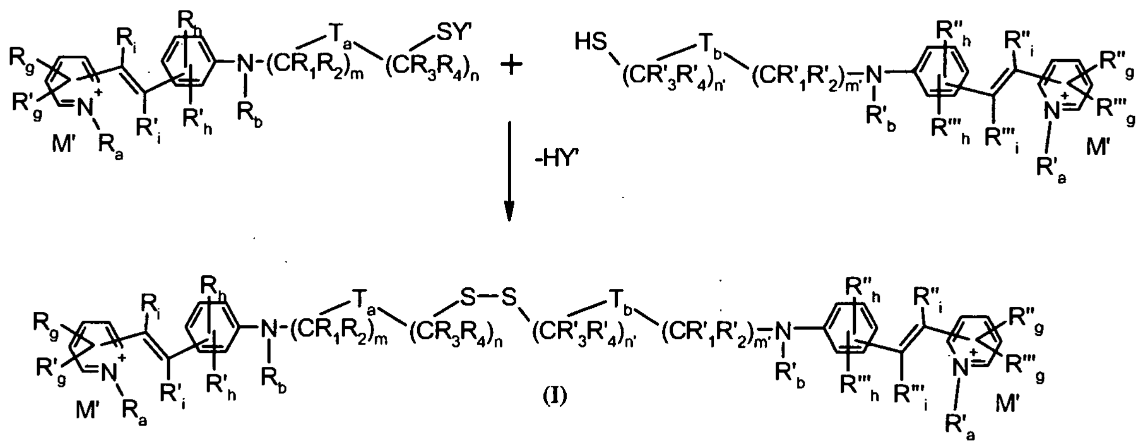


avec $R_a$, $R'_a$, $R_b$, $R'_b$, $R_g$, $R'_g$, $R''_g$, $R''_g$, $R_h$, $R'_h$, $R''_h$, $R'''_h$, $R_i$, $R'_i$, $R''_i$, $R'''_i$, $R'_1$, $R_2$, $R'_2$, $R_3$, $R'_3$, $R_4$, $R'_4$, m, m', n, n', $T_a$, $T_b$ et M' sont tels que définis précédemment ; Y' représente un groupement protecteur de fonction thiol.

La composition de l'invention contient au moins un colorant fluorescent de formule (I) ou (II). Outre la présence d'au moins un colorant fluorescent thiol de formule (I), la composition de l'invention peut également contenir un agent réducteur. Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol. Cet agent réducteur peut également être choisi parmi les borohydrures et leurs dérivés, comme par exemple les sels du borohydrure, du cyanoborohydrure, du triacétoxyborohydrure, du triméthoxy-borohydrure : sels de sodium, lithium, potassium, calcium, ammoniums quaternaires (tétraméthylammonium, tétraéthylammonium, tétra n-butylammonium, benzyltriéthyl-ammonium) ; le catécholborane.

La composition tinctoriale utile dans l'invention contient en général une quantité de colorants fluorescents de formule (I) ou (II) comprise entre 0,001 et 50 % par rapport au poids total de la composition. De préférence, cette quantité est comprise entre 0,005 et 20 % en poids et encore plus préférentiellement entre 0,01 et 5 % en poids par rapport au poids total de la composition.

La composition tinctoriale peut en outre contenir des colorants directs additionnels. Ces colorants directs sont par exemple choisis parmi les colorants directs nitrés benzéniques neutres, acides ou cationiques, les colorants directs azoïques neutres, acides ou cationiques, les colorants tétraazapentaméthiniques, les colorants quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La composition tinctoriale peut contenir une ou plusieurs bases d'oxydation et/ou un ou plusieurs coupleurs conventionnellement utilisés pour la teinture de fibres kératiniques.

Parmi les bases d'oxydation, on peut citer les para-phénylènediamines, les bisphénylalkylènediamines, les para-aminophénols, les bis-para-aminophénols, les ortho-aminophénols, les bases hétérocycliques et leurs sels d'addition

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

Le ou les coupleurs sont chacun généralement présents en quantité comprise entre 0,001 et 10 % en poids du poids total de la composition tinctoriale, de préférence entre 0,005 et 6%.

La ou les bases d'oxydation présentes dans la composition tinctoriale sont en général présentes chacune en quantité comprise entre 0,001 à 10 % en poids du poids total de la composition tinctoriale, de préférence entre 0,005 et 6 % en poids.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que les hydroxydes de métal alcalin comme la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants lorsqu'ils sont présents sont, de préférence présents dans des proportions de préférence comprises entre 1 et 40 % en poids environ par rapport au poids total de la composition tinctoriale, et encore plus préférentiellement entre 5 et 30 % en poids environ.

La composition tinctoriale peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non tels que les silicones aminés, des agents filmogènes, des céramides, des agents conservateurs, des agents opacifiants, des polymères conducteurs.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière

telle que les propriétés avantageuses attachées intrinsèquement à la composition de teinture conforme à l'invention ne soient pas, ou substantiellement pas, altérées par la ou les adjonctions envisagées.

Le pH de la composition tinctoriale est généralement compris entre 3 et 14 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (γ) suivante :

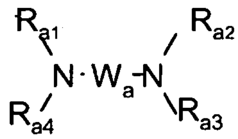

(γ)

dans laquelle $W_a$ est un reste propylène éventuellement substitué par un groupement hydroxy ou un radical alkyle en $C_1$-$C_4$ ; $R_{a1}$, $R_{a2}$, $R_{a3}$ et $R_{a4}$, identiques ou différents, représentent un atome d'hydrogène, un radical alkyle en $C_1$-$C_4$ ou hydroxyalkyle en $C_1$-$C_4$.

La composition tinctoriale peut se présenter sous des formes diverses, telles que sous forme de liquide, de crème, de gel, ou sous toute autre forme appropriée pour réaliser une teinture des fibres kératiniques, et notamment des cheveux.

Le procédé de coloration de l'invention consiste à appliquer la composition de l'invention sur les matières kératiniques, en particulier les fibres kératiniques, notamment humaines telles que les cheveux, plus particulièrement les cheveux foncés, la composition tinctoriale de l'invention comprenant au moins un colorant fluorescent de formule (I) ou (II).

Selon un mode de réalisation particulier dans le procédé de l'invention un agent réducteur peut être appliqué en pré-traitement avant l'application de la composition contenant au moins un colorant fluorescent de formule (I) ou (II).

Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol. Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catécholborane.

Ce prétraitement peut être de courte durée, notamment de 0,1 seconde à 30 minutes, de préférence de 1 minute à 15 minutes avec un agent réducteur tel que cité précédemment.

Selon un autre procédé, la composition comprenant au moins un colorant fluorescent de formule (I) ou (II) contient également au moins un agent réducteur tel que défini précédemment. Cette composition est alors appliquée aux cheveux.

Lorsque le colorant fluorescent (I) ou (II) comprend un groupement Y protecteur de la fonction thiol, le procédé de l'invention peut être précédé d'une étape de déprotection visant à restituer *in-situ* la fonction SH.

A titre d'exemple il est possible de déprotéger la fonction S-Y avec Y groupement protecteur en ajustant le pH comme suit :

| Y : groupe protecteur | déprotection |
|---|---|
| | |
| alkylcarbonyle, | pH>9 |
| arylcarbonyle, | pH>9 |
| alcoxycarbonyle, | pH>9 |
| aryloxycarbonyle, | pH>9 |
| arylalcoxycarbonyle | pH>9 |

(suite)

| Y : groupe protecteur | déprotection |
|---|---|
| (di)(alkyl)aminocarbonyle, | pH>9 |
| (alkyl)arylaminocarbonyle | pH>9 |
| aryle éventuellement substitué tel que le phényle, | pH>9 |
| hétéroaryle monocyclique à 5, 6 ou 7 chaînons tel que l'oxazolium ; | pH>9 |
| Hétéroaryle bicyclique à 8 à 11 chaînons tels que benzoimidazolium, ou benzoxazolium | pH>9 |

L'étape de déprotection peut également être réalisée au cours d'une étape de prétraitement des cheveux comme par exemple, le prétraitement réducteur des cheveux.

Selon un autre procédé de coloration, la composition comprenant au moins un colorant fluorescent de formule (I) ou (II) contient également au moins un agent réducteur tel que défini précédemment. Cette composition est alors appliquée aux cheveux.

Selon une variante, l'agent réducteur est ajouté à la composition tinctoriale contenant au moins un colorant fluorescent (I) ou (II) au moment de l'emploi.

Selon une autre variante, l'agent réducteur est appliqué en post-traitement, après l'application de la composition contenant au moins un colorant fluorescent (I) ou (II). La durée du post traitement avec l'agent réducteur peut être courte, par exemple de 0,1 seconde à 30 minutes de préférence de 1 minute à 15 minutes, avec un agent réducteur tel que décrit précédemment. Selon un mode de réalisation particulier l'agent réducteur est un agent de type thiol ou borohydrure tel que décrit précédemment.

Un mode de réalisation particulier de l'invention concerne un procédé dans lequel le colorant fluorescent de formule (I) ou (II) peut être appliqué directement aux cheveux sans réducteurs, exempt de pré ou post-traitement réducteurs.

Un traitement avec un agent oxydant peut éventuellement être associé. On pourra utiliser n'importe quel type d'agent oxydant classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons tels que les uricases et les oxygénases à 4 électrons comme les laccases. L'utilisation du peroxyde d'hydrogène est particulièrement préférée.

Cet agent oxydant peut être appliqué sur les fibres avant ou après l'application de la composition contenant au moins un colorant fluorescent de formule (I) ou (II).

L'application de la composition tinctoriale selon l'invention est généralement effectuée à température ambiante. Elle peut cependant être réalisée à des températures variant de 20 à 180°C.

L'invention a aussi pour objet un dispositif à plusieurs compartiments ou "kit" de teinture dans lequel un premier compartiment renferme une composition tinctoriale comprenant au moins un colorant fluorescent de formule (I) ou (II) et un deuxième compartiment renferme un agent réducteur capable de réduire les fonctions disulfures des matières kératiniques.

L'un de ces compartiments peut en outre contenir un ou plusieurs autres colorants de type colorant direct ou colorant d'oxydation.

Elle concerne aussi un dispositif à plusieurs compartiments dans lequel un premier compartiment contient une composition tinctoriale comprenant au moins un colorant fluorescent de formule (I) ou (II) ; un deuxième compartiment contient un agent réducteur capable de réduire la liaison disulfure des matières kératiniques ; un troisième compartiment contient un agent oxydant.

Une autre variante concerne un dispositif à plusieurs compartiment ou « kit » de teinture comprenant un premier compartiment qui contient une composition avec au moins un colorant fluorescent protégé de formule (II) et un deuxième compartiment comprenant une composition contenant un agent de déprotection telle qu'une base.

Chacun des dispositifs mentionnés ci-dessus peut être équipé d'un moyen permettant de délivrer sur les cheveux le mélange souhaité, par exemple tel que les dispositifs décrits dans le brevet FR2 586 913.

Les exemples qui suivent servent à illustrer l'invention sans toutefois présenter un caractère limitatif. Les colorants fluorescents des exemples ci-après ont été entièrement caractérisés par les méthodes spectroscopiques et spectrométriques classiques.

**EXEMPLES**

**EXEMPLES DE SYNTHESE**

**<u>Exemple 1 :</u> Synthèse du sel de 4-((E)-2-{4-[(4,13-diméthyl-17-{4-[(E)-2-(1-méthylpyridinium-4-yl)vinyl]phényl}-5,12-dioxo-8,9-dithia-4,13,17-triazaoctadéc-1-yl)(méthyl)amino]phényl}vinyl)-1-méthylpyridinium [1]**

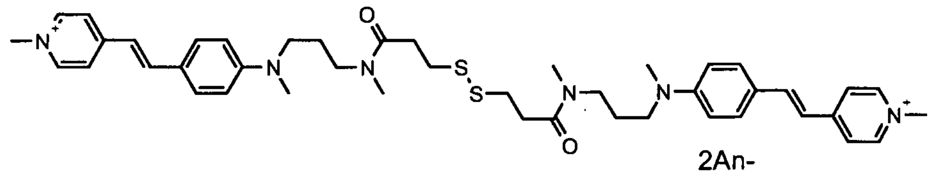

**[1]**

## Schéma de synthèse

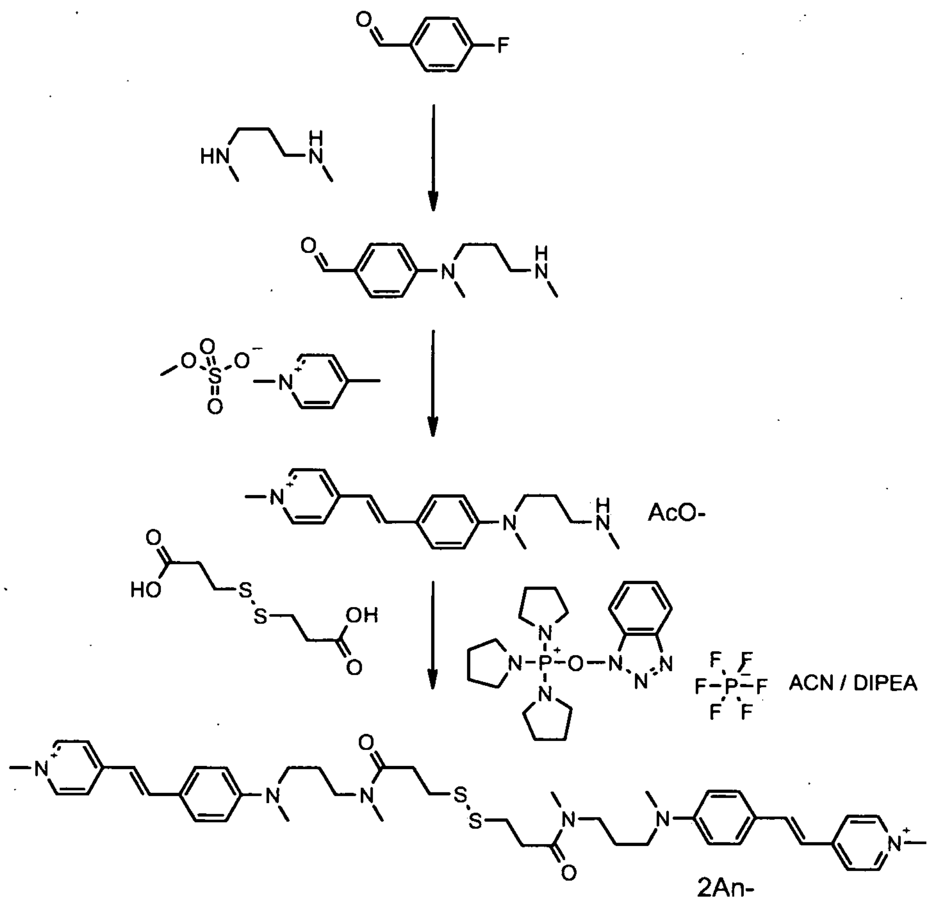


**[1]**

**Etape 1 : Synthèse du 4-{méthyl[3-(méthylamino)propyl]amino} benzaldéhyde** 8,64 mL de 4-fluorobenzaldéhyde, 13,36 g de carbonate de potassium et 30 mL de N-méthylpyrrolidinone (NMP) sont mélangés sous agitation et chauffage à 110 ˚C. 11,9 mL de 1,3-bis(méthylamino)propane sont ajoutés goutte à goutte au milieu réactionnel maintenu sous agitation et chauffage à 110 ˚C pendant 3 jours. Le mélange réactionnel est concentré et 54 g d'huile recueillis.

**Etape 2 : Synthèse de l'acétate de 1-méthyl-4-[(E)-2-(4-{méthyl[3-(méthylamino)-propyl]amino}phényl)vinyl] pyridinium**

25 g de 4-{méthyl[3-(méthylamino)propyl]amino}benzaldehyde en solution dans 30 mL d'isopropanol et 10.9 mL de pyrrolidine sont agités à température ambiante pendant 10 min. 7,6 mL d'acide acétique sont ajoutés au milieu réactionnel maintenu sous agitation à 20 ˚C pendant 20 min. 26,55 g de méthylsulfate de 1,4-diméthylpyridinium dilués dans 20 mL d'isopropanol sont additionnés. Le milieu réactionnel est maintenu sous agitation à température ambiante pendant 5 jours. Le mélange obtenu est extrait 3 fois au dichlorométhane (DCM), concentré et purifié par chromatographie liquide eau/butanol. Après concentration sous vide des fractions pures, 3,4 g de poudre rouge sont recueillis.

**Etape 3 : Synthèse du sel de 4-((E)-2-{4-[(4,13-diméthyl-17-{4-[(E)-2-(1-méthylpyridinium-4-yl)vinyl]phényl}-5,12-dioxo-8,9-dithia-4,13,17-triazaoctadec-1-yl)(méthyl)amino]phenyl}vinyl)-1-méthylpyridinium**

1,63 g d'acétate de 1-méthyl-4-[(E)-2-(4-{méthyl[3-(méthylamino)propyl]amino}phényl)-vinyl]pyridinium sont solubilisés dans 5 mL d'acétonitrile (ACN). 600 μL de diisopropyléthylamine (DIPEA), 420 mg d'acide 3,3'-dithiopropanoïque puis 1,09 g d'héxafluorophosphate de (1H-1,2,3-benzotriazol-1-yloxy)(tripyrrolidin-1-yl)-phosphonium sont ajoutés au mélange. Après 24 h d'agitation à température ambiante, le mélange est concentré et purifié par chromatographie sur gel de silice (phase inverse greffée $C_{18}$ ; élution eau/ACN/acide trifluoroacétique 0,1 %). Les fractions pures sont réunies et neutralisées par ajout d'hydrogénocarbonate de sodium, concentrées sous vide et le solide obtenu est repris dans un mélange acétone/ACN. Après filtration, l'évaporation des solvants conduit à un solide rouge de masse 1,77 g. Les analyses indiquent que le produit obtenu est conforme à la structure attendue. Les contres ions An- sont constitués d'un mélange de trifluoroacétate et hydrogénocarbonate. LCMS : ions positifs m/z = 383, 256 et 192 correspondant respectivement à $[M/2]^{2+}$, $[M+H]^{3+}$ et $[M+2H]^{4+}$. $\lambda_{max}$ 468 nm.

<u>Exempté 2 :</u> **Synthèse du sel de 1-méthyl-4-{(E)-2-[4-(méthyl{3-[méthyl(3-sulfanylpropanoyl)-amino]propyl} amino)phényl]vinyl}pyridinium [2]**

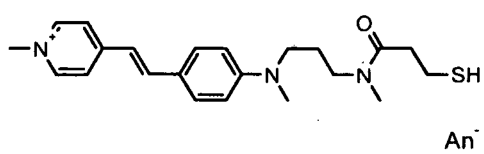

**[2]**

**Schéma de synthèse**

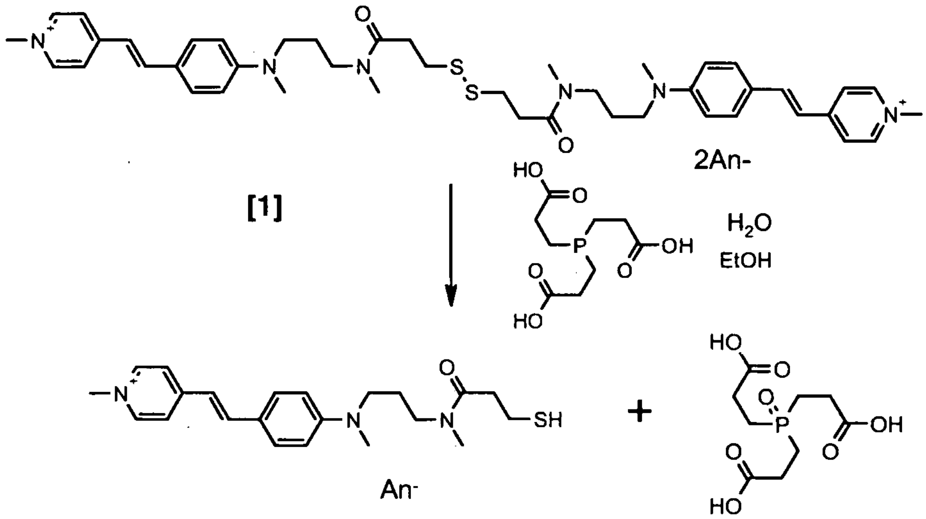


2An-

[1]

An⁻

**[2]** An⁻ = mélange de TFA⁻ et $HCO_3^-$

**Etape 1 : Synthèse du sel de 1-méthyl-4-{(E)-2-[4-(méthy){3-[méthyl(3-sulfanyl-propanoyl)amino]propyl}amino) phényl]vinyl}pyridinium [2]**

60 mg du sel de 1-méthyl-4-{(E)-2-[4-(méthyl{3-[méthyl(3-sulfanylpropanoyl)-amino]propyl}amino)phényl]vinyl}pyridinium sont dissous dans 10 mL de mélange eau/éthanol 1 : 1 ; 2 éq. d'hydrate de chlorhydrate d'acide 3-[bis (2-carboxy-éthyl)phosphino]propanoïque en solution dans 1 mL d'eau et 4 éq de bicarbonate de sodium en solution dans 1 mL d'eau sont ajoutés au mélange. Après 30 minutes d'agitation à 40°C sous atmosphère inerte, les analyses indiquent que le mélange contient très majoritairement le produit attendu **[2].**

Analyse LC-MS : LC-DAD (400-700 nm)
Colonne : Waters XTerra MS C18 5µm (4.6 x 50) mm
Phase Mobile : A : Eau + acide formique 0.1% / B : ACN
Gradient linéaire : T (min) A%/B% : 0 min 95/5 ; 8 min 0/100
Débit : 1mL/min.
Détection : UV Barrette de diodes λ=400-700nm
MS (ESI+) : correspond au pic de masse du monocation du produit attendu [2]

**EXEMPLES DE COLORATION**

**Exemple 1 : Procédé de coloration - composé [1]**

Préparation d'une composition A

| | |
|---|---|
| Colorant disulfure [1] | $10^{-3}$ mol% |
| Alcool Benzylique | 4 g |
| Polyéthylèneglycol 6OE | 6 g |
| Hydroxyethylcellulose | 0,7 g |
| Alkylpolyglucoside en solution aqueuse à 65%MA | 4.5 g |
| Eau déminéralisée | qsp 100g |

Préparation d'une composition B

| | |
|---|---|
| Acide thioglycolique | 1M |
| Hydroxyde de Sodium | qsp pH 8,5 |
| Eau déminéralisée | qsp 100g |

Au moment de l'emploi, on mélange les compositions A (9 mL) et B (1 mL), puis on applique le mélange obtenu sur une mèche de 1 g de cheveux foncés (hauteur de ton 4) pendant 30 minutes à température ambiante (les mèches sont retournées et réimprégnées après 15 minutes).

Après rinçage à l'eau courante et séchage, on observe un éclaircissement des cheveux ainsi traités : la mèche de hauteur de ton 4 est devenue visuellement plus claire que des mèches témoins non traitées.

**<u>Exemple 2</u> : procédé de coloration avec le composé [2]**

10 mL de solution fraîche du composé **[2]** de l'exemple de synthèse sont appliqués sur une mèche de 1g cheveux de hauteur de ton 4 disposées au fond d'un bol pendant 30 minutes à température ambiante (les mèches sont retournées et réimprégnées après 15 minutes).

Les mèches sont ensuite rincées à l'eau courante et séchées.

Après coloration, la mèche de hauteur de ton 4 est devenue visuellement plus claire que des mèches témoins non traitées.

**Rémanence vis-à-vis des shampooings successifs :**

Les mèches ainsi traitées sont divisées en deux, une moitié est soumise à 5 shampooings successifs selon un cycle qui comprend le mouillage des mèches à l'eau, le lavage avec un shampoing classique, un rinçage à l'eau suivi d'un séchage.

*Observations visuelles*

Lors des shampooings, il n'y a pas de dégorgement visible, la mousse des shampooings et les eaux de rinçage ne sont pas colorées

La couleur observée est conservée et l'effet d'éclaircissement reste visible sur les cheveux de hauteur de ton 4 ainsi traités.

*Résultats dans le système L *a*b* :*

La couleur des mèches avant et après les 5 lavages a été évaluée dans le système L*a*b* au moyen d'un spectrophotomètre CM 2600D MINOLTA ®, (Illuminant D65).

Dans ce système L* a* b*, L* représente la luminosité, a* indique l'axe de couleur vert/rouge et b* l'axe de couleur bleu/jaune. Plus la valeur de L est élevé, plus la couleur est claire ou peu intense. Inversement, plus la valeur de L est faible, plus la couleur est foncée ou très intense. Plus la valeur de a* est élevée plus la nuance est rouge et plus la valeur de b* est élevée plus la nuance est jaune.

La variation de la coloration entre les mèches de cheveux HT4, colorées et lavées est mesurée par (ΔE) selon l'équation suivante :

$$\Delta E = \sqrt{(L^* - L_o{}^*)^2 + (a^* - a_o{}^*)^2 + (b^* - b_o{}^*)^2}$$

Dans cette équation, L*, a* et b* représentent les valeurs mesurées avant coloration et $L_0$*, $a_0$* et $b_0$* représentent les valeurs mesurées avant coloration (ou shampooing).

Plus la valeur de ΔE est importante, plus la différence de couleur entre les mèches HT4 et les mèches colorées est importante.

| Composé | L* | a* | b* | Δ E |
|---|---|---|---|---|
| Référence mèche de HT4 | 17,27 | 2,78 | 3,19 | |
| Composé 1 | 19,36 | 8,32 | 9,20 | 8,45 |
| Composé 1 - 5 shampooings | 19,58 | 8,37 | 9,09 | 8,45 |

Les résultats du tableau ci-dessus montrent que la coloration évolue très peu après 5 shampooings. Ainsi la coloration et l'effet éclaircissant sur les cheveux restent quasiment inchangés ce qui montre une très bonne résistance aux shampooings des colorants de l'invention.

*Résultats de Réflectance :*

Les performances d'éclaircissement des compositions conformes à l'invention et leur rémanence vis-à-vis de shampooings successifs ont été exprimées en fonction de la réflectance des cheveux. Ces réflectances sont comparées à la réflectance d'une mèche de cheveux non traitées de hauteur de ton HT4 .

La réflectance est mesurée au moyen d'un appareil spectrophotocolorimètre KONIKA-MINOLTA ®, CM 2600d et après irradiation des cheveux avec une lumière visible dans la gamme de longueurs d'onde allant de 400 à 700 nanomètres, (voir figure 1).

On constate tout d'abord que la réflectance d'une mèche de cheveux traitée avec une composition selon l'invention est supérieure à celle des cheveux non traités. Les mèches traitées apparaissent donc plus claires.

De plus, les résultats montrent que la réflectance des mèches de cheveux de hauteur de ton 4 traitées avec la composition de l'invention évoluent très peu après 5 shampooings. Ainsi la coloration et l'effet éclaircissant sur les cheveux restent quasiment inchangés ce qui montre une très bonne résistance aux shampooings des colorants de l'invention.

## Revendications

**1.** Colorant fluorescent de formule (I) ou (II) suivante :

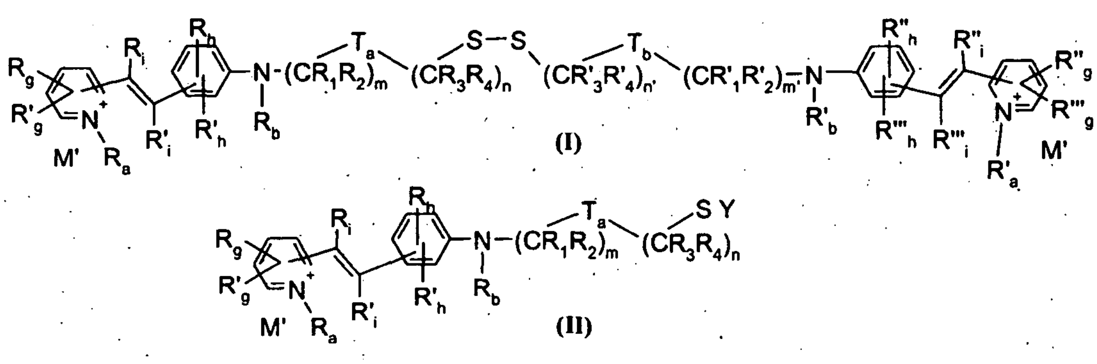

(I)

(II)

ses sels d'acide organique ou minéral, isomères optiques, isomères géométriques, et les solvates tels que hydrates ; formules (I) ou (II) dans lesquelles :

- ➢ $R_a$ et $R'_a$, identiques ou différents, représentent un groupement aryl($C_1$-$C_4$)alkyle ou un groupement ($C_1$-$C_6$) alkyle éventuellement substitué par un groupement hydroxy ou amino, $C_1$-$C_4$ alkylamino, ($C_1$-$C_4$) dialkylamino, lesdits radicaux alkyle pouvant former avec l'atome d'azote qui les portent un hétérocycle comprenant de 5 à 7 chaînons, comprenant éventuellement un autre hétéroatome différent ou non de l'azote ;
- ➢ $R_b$ et $R'_b$, identiques ou différents, représentent un atome d'hydrogène, un groupement aryl($C_1$-$C_4$)alkyle ou un groupement ($C_1$-$C_6$) alkyle éventuellement substitué ;
- ➢ $R_g$, $R'_g$, $R''_g$ et $R'''_g$, identiques ou différents, représentent un atome d'hydrogène, un groupement amino, ($C_1$-$C_4$) alkylamino, ($C_1$-$C_4$) dialkylamino, trifluorométhyle, un radical acylamino, ($C_1$-$C_4$)alcoxy ($C_1$-$C_4$)alkylcarbonyloxy, ($C_1$-$C_4$)alcoxycarbonyle, ($C_1$-$C_4$)alkylcarbonylamino, ($C_1$-$C_4$)alkylsulfonylamino, un radical

$(C_1-C_3)$alkyle ;

➢ $R_h$, $R'_h$, $R''_h$ et $R'''_n$, identiques ou différents, représentent un atome d'hydrogène, un atome d'halogène, un groupement dialkylamino $(C_1-C_4)$, $(C_1-C_4)$alkylcarbonylamiho, un radical acylamino, $(C_1-C_4)$alkylsulfonylàmino, ou un radical $(C_1-C_4)$ alkyle ;

➢ ou alors deux groupements $R_g$ et $R'_g$; $R''_g$ et $R'''_g$; $R_h$ et $R'_h$; $R''_h$ et $R'''_h$ portés par deux atomes de carbone adjacents, forment ensembles un cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, $(C_1-C_4)$alkylamino, $(C_1-C_4)$dialkylamino, cyano, carboxy, hydroxy, trifluorométhyle, acylamino, alcoxy en $C_1$-$C_4$, (poly)hydroxyalcoxy en $C_2$-$C_4$, $(C_1-C_4)$alkylcarbonyloxy $(C_1-C_4)$alcoxycarbonyle, $(C_1-C_4)$alkylcarbonylamino, un radical acylamino, carbamoyle, $(C_1-C_4)$alkylsulfonylamino, un radical aminosulfonyle, ou un radical $(C_1-C_{16})$alkyle éventuellement substitué par un groupement choisi parmi $(C_1-C_{12})$ alcoxy, hydroxy, cyano, carboxy, amino, $(C_1-C_4)$alkylamino et $(C_1-C_4)$dialkylamino, ou alors les deux radicaux alkyle portés par l'atome d'azote du groupement amino forment un hétérocycle comprenant de 5 à 7 chaînons et comprenant éventuellement un autre hétéroatome identique ou différent à celui de l'atome d'azote ;

➢ $R_1$, $R'_i$, $R''_i$ et $R'''_i$, identiques ou différents, représentent un atome d'hydrogène, ou un groupement $(C_1-C_4)$ alkyle ;

➢ $R_1$, $R_2$, $R_3$, $R_4$, $R'_1$, $R'_2$, $R'_3$ et $R'_4$, identiques ou différents, représentent un atome d'hydrogène ou un groupement $(C_1-C_4)$ alkyle, $(C_1-C_{12})$alcoxy, hydroxy, cyano, carboxy, (di)$(C_1-C_4)$(alkyl)amino, lesdits radicaux alkyle pouvant former avec l'atome d'azote qui les portent un hétérocycle comprenant de 5 à 7 chaînons, comprenant éventuellement un autre hétéroatome différent ou non de l'azote ;

➢ $T_a$ et $T_b$, identiques ou différents, représentent la combinaisons des radicaux choisis parmi $-SO_2-$, -O-, -S-, -N(R)-, -CO-, avec R, représente un atome d'hydrogène, un radical alkyle en $C_1$-$C_4$, hydroxyalkyle en $C_1$-$C_4$; ou un aryl$(C_1-C_4)$alkyle ;

➢ m, m', n et n', identiques ou différents, représentent un entier compris inclusivement entre 0 et 6 avec m+n et m'+n', identiques ou différents, représentent un entier compris inclusivement entre 1 et 10 ;

➢ M' représentant un contre-ion anionique ; et

➢ Y représente : i) un atome d'hydrogène ; ii) un métal alcalin ; iii) un métal alcalino-terreux ; iv) un groupement ammonium : $N^+R^\alpha R^\beta R^\gamma R^\delta$ ou un groupement phosphonium : $P^+R^\alpha R^\beta R^\gamma R^\delta$ avec $R^\alpha$, $R^\beta$, $R^\gamma$ et $R^\delta$, identiques ou différents, représentant un atome d'hydrogène ou un groupe $(C_1-C_4)$alkyle ; ou v) un groupement protecteur de fonction thiol ;

étant entendu que lorsque le composé de formule (I) ou (II) contient d'autres parties cationiques, il se trouve associé à un ou plusieurs contre-ions anioniques permettant d'atteindre l'électroneutralité de la formule (I) ou (II).

2. Colorant fluorescent (I) ou (II) selon la revendication 1 dans lequel lorsque $T_a$ et $T_b$ représentent -C(O)-N(R)- ou -N(R)-C(O)-.

3. Colorant fluorescent de formule (II) selon une des revendications 1 ou 2 dans lequel Y représente un atome d'hydrogène, ou un métal alcalin.

4. Colorant fluorescent de formule (II) selon une des revendications 1 ou 2 dans lequel Y représente un groupement protecteur.

5. Colorant fluorescent de formule (I) selon la revendication précédente dans lequel Y représente un groupement protecteur choisi parmi les radicaux suivants :

➢ $(C_1-C_4)$alkylcarbonyle ;
➢ $(C_1-C_4)$alkylthiocarbonyle ;
➢ $(C_1-C_4)$alcoxycarbonyle ;
➢ $(C_1-C_4)$alcoxythiocarbonyle ;
➢ $(C_1-C_4)$alkylthio-thiocarbonyle ;
➢ (di) $(C_1-C_4)$ (alkyl)aminocarbonyle ;
➢ (di) $(C_1-C_4)$ (alkyl)aminothiocarbonyle;
➢ arylcarbonyle ;
➢ aryloxycarbonyle ;
➢ aryl$(C_1-C_4)$alkcoxycarboiiyle ;
➢ (di) $(C_1-C_4)$ (alkyl)aminocarbonyle ;
➢ $(C_1-C_4)$(alkyl)arylaminocarbonyle ;

➢ carboxy;
➢ $SO_3^-$ ; $M^+$ avec $M^+$ représentant un métal alcalin ou alors M' de la formule (II) et $M^+$ sont absents ;
➢ aryle éventuellement substitué,
➢ hétéroaryle éventuellement substitué;
➢ hétérocycloalkyle éventuellement substitué, éventuellement cationique ;
➢ isothiouronium $-C(NR'^cR'^d)=N^+R'^eR'^f$, $An^-$ avec $R'^c$, $R'^d$, $R'^e$ et $R'^f$, identiques ou différents représentent un atome d'hydrogène ou un groupement alkyle ; préférentiellement $R'^c$ à $R'^f$ représentent un atome d'hydrogène ; et $An^-$ représente un contre-ion anionique ;
➢ isothiourée $-C(NR'^cR'^d)=NR'^e$ ; avec $R'^c$, $R'^d$ et $R'^e$ tels que définis précédemment ;
➢ (di)arylalkyle éventuellement substitué ;
➢ (di)hétéroarylakyle éventuellement substitué ;
➢ $CR'^1R'^2R'^3$ avec $R'^1$, $R'^2$ et $R'^3$ identiques ou différents, représentant un atome d'halogène ou un groupe choisi parmi :

■ alkyle ;
■ alcoxy ;
■ aryle éventuellement substitué ;
■ hétéroaryle éventuellement substitué ;

➢ $P(Z^1)R''^1R''^2R''^3$ avec $R''^1$, et $R''^2$ identiques ou différents représentent un groupement hydroxy, alcoxy ou alkyle, $R''^3$ représente un groupement hydroxy ou alcoxy, et $Z^1$ représente un atome d'oxygène ou de soufre ;
➢ cyclique stériquement encombré ; et
➢ alcoxyalkyle éventuellement substitué.

**6.** Colorant fluorescent selon l'une quelconque des revendications précédentes dans lequel Y représente un métal alcalin ou un groupement protecteur choisi parmi :

➢ $(C_1-C_4)$alkylcarbonyle ;
➢ arylcarbonyle ;
➢ $(C_1-C_4)$alcoxycarbonyle ;
➢ aryloxycarbonyle ;
➢ aryl$(C_1-C_4)$alcoxycarbonyle ;
➢ (di) $(C_1-C_4)$ (alkyl)aminocarbonyle ;
➢ $(C_1-C_4)$(alkyl)arylaminocarbonyle ;
➢ aryle éventuellement substitué ;
➢ hétéroaryle monocyclique à 5 ou 6 chaînons ;
➢ hétéroaryle bicyclique cationique à 8 à 11 chaînons tels que benzoimidazolium, ou le benzoxazolium ; ces groupements étant éventuellement substitué par un ou plusieurs, identiques ou différents, groupes $(C_1-C_4)$ alkyle ;
➢ hétérocycle cationique de formule suivante :

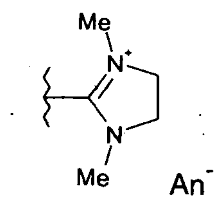

➢ isothiouronium $-C(NH_2)=N^+H_2$; $An^-$;
➢ isothiourée $-C(NH_2)=NH$ ; et
➢ $SO_3^-$, $M^+$ avec $M^+$ représentant un métal alcalin ou alors M' de la formule (II)

et $M^+$ sont absents.

**7.** Colorant fluorescent selon l'une quelconque des revendications précédentes, choisi parmi les colorants fluorescents de formule **(Ia)** ou **(IIa)**, possédant chacun un groupement éthylène qui relie la partie pyridinium au phényle en position ortho ou para soit en positions 4-4', 4-2' ou 2-4' :

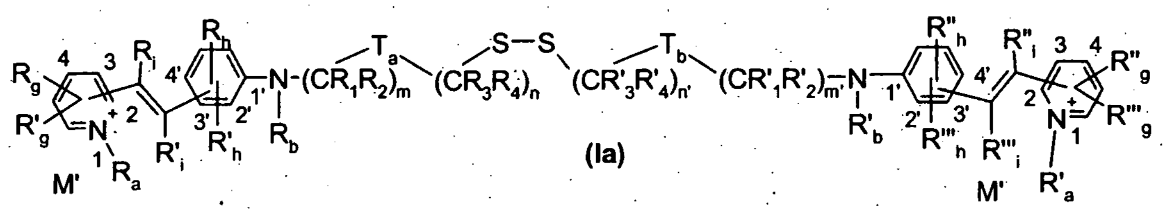

(Ia)

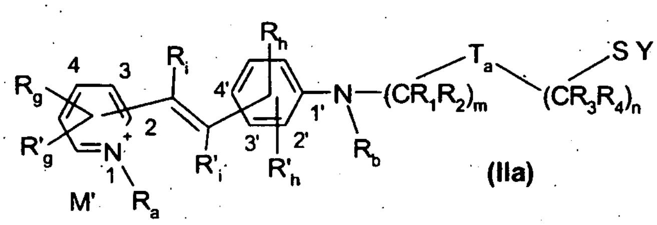

(IIa)

formule (Ia) et (IIa) avec $R_1$, $R_2$, $R_3$, $R_4$, $R_a$, $R_b$, $R_i$, $R'_i$, $R_g$, $R'_g$, $R_h$, $R'_h$, m, n, Y et $R'_1$, $R'_2$, $R'_3$, $R'_4$, $R'_a$, $R'_b$, $R''_1$, $R'''_i$, $R''_g$, $R'''_g$, $R''_h$, $R'''_h$, m' et n' sont tels que définis dans une quelconque des revendications précédentes.

**8.** Colorant fluorescent de formule (I) ou (Ia) selon une des revendications 1 ou 7 qui est symétrique.

**9.** Colorant fluorescent selon l'une quelconque des revendications précédentes, choisi parmi les colorant suivants :

| | |
|---|---|
| 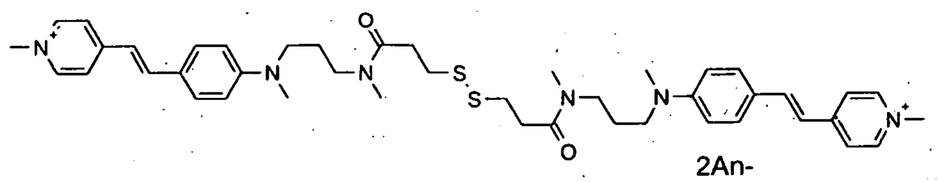 2An- | |
| 1 | |
| 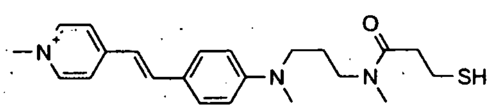 An⁻ | 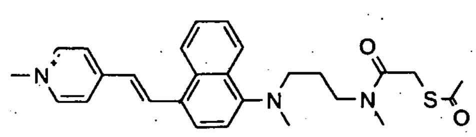 An |
| 2 | 3 |
| 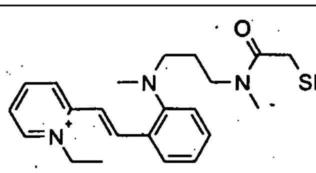 An⁻ | 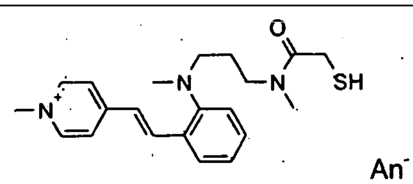 An⁻ |
| 3 | 4 |
| 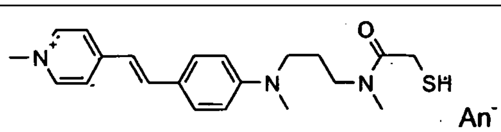 An⁻ | 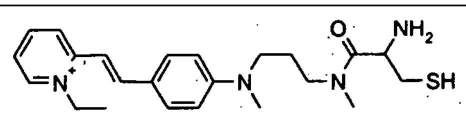 |
| 5 | 6 |
| 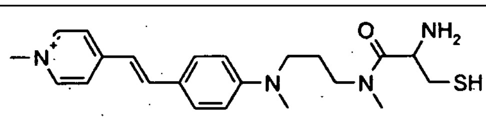 An⁻ | 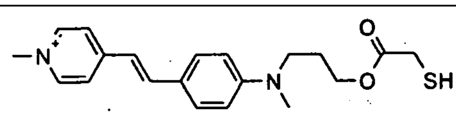 An⁻ |

(suite)

| | |
|---|---|
| **7** | **8** |
| 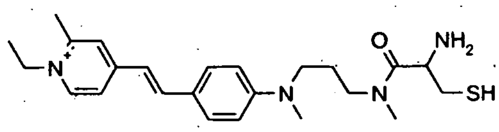 $An^-$ | 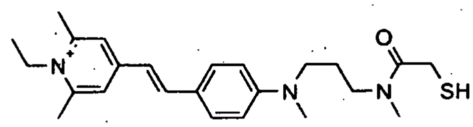 $An^-$ |
| **9** | **10** |
| 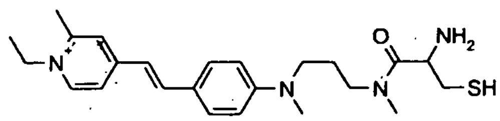 $An^-$ | 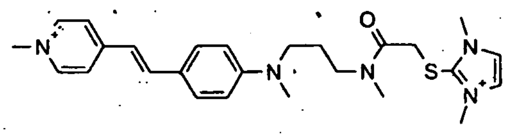 $2An^-$ |
| **11** | **12** |
| 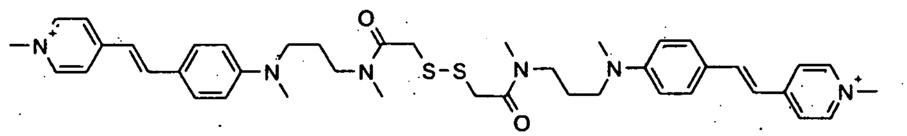 2M' | |
| **13** | |
| 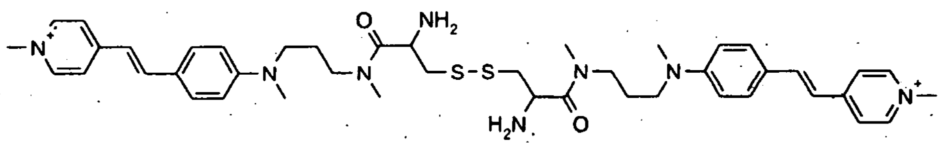 2M' | |
| **14** | |
| 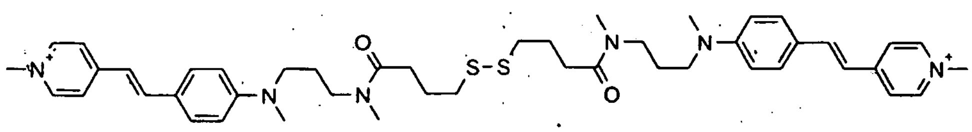 2M' | |
| **15** | |
| 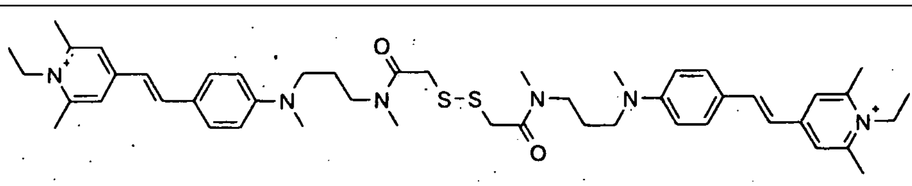 2M' | |
| **16** | |
| 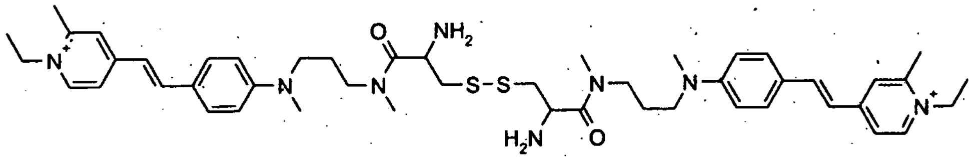 2M' | |
| **17** | |
| 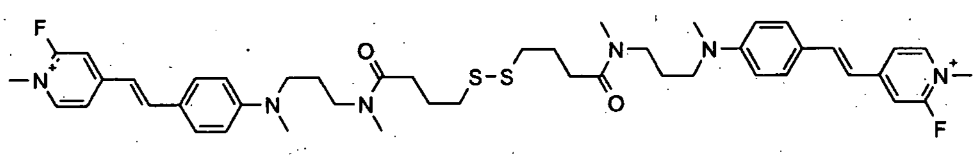 2M' | |
| **18** | |

(suite)

| |
|---|
| 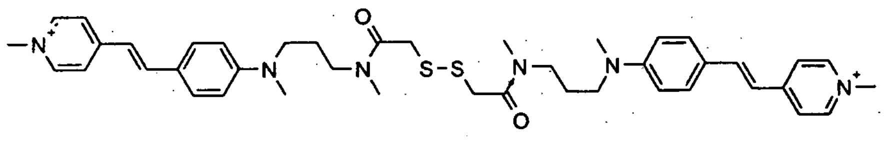 2M' |
| **19** |
| 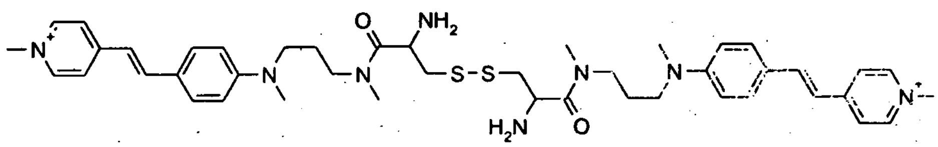 2M' |
| **20** |
| 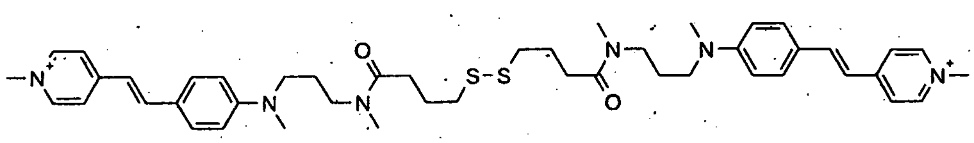 2M' |
| **21** |
| 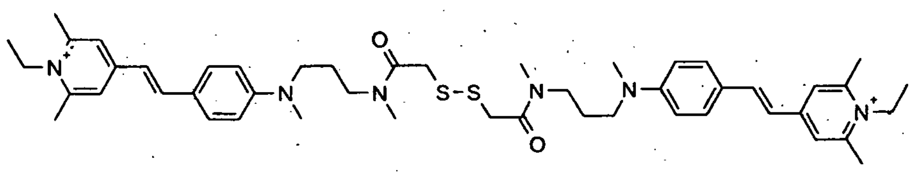 2M' |
| **22** |
| 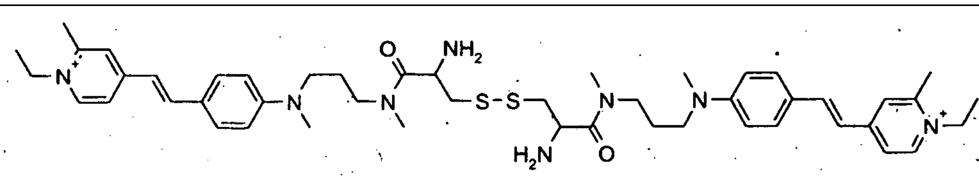 2M' |
| **23** |
| 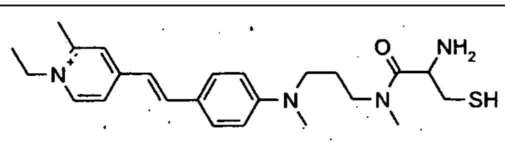 $An^-$ |
| **24** |
| 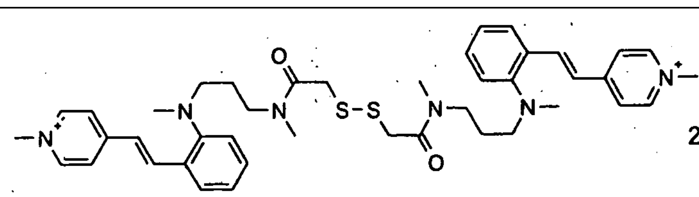 2M' |
| **25** |
| 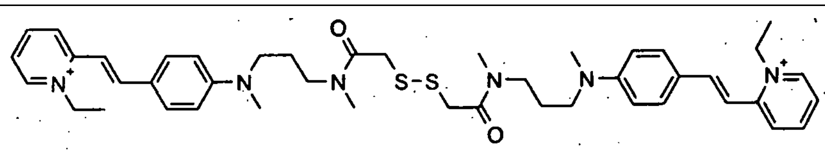 2M' |
| **26** |

(suite)

| |
|---|
| 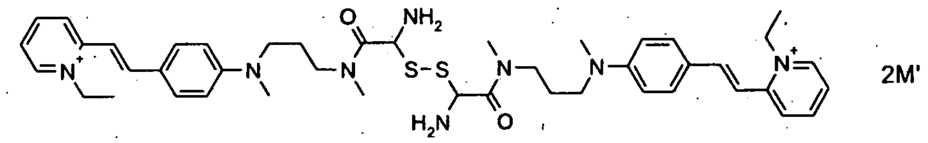 |
| **27** |
| 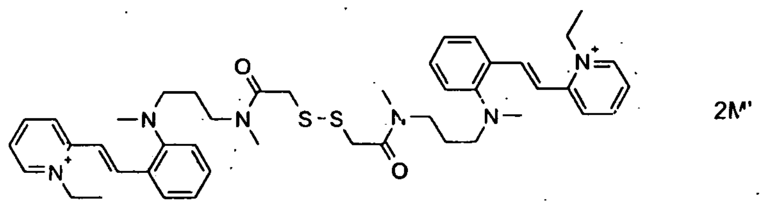 |
| **28** |

avec M', An⁻, identiques ou différents, représentant un contre-ion.anionique.

**10.** Composition tinctoriale comprenant dans un milieu cosmétique approprié, un colorant fluorescent de formule (I) ou (II) tel que défini dans une quelconque des revendications 1 à 9.

**11.** Composition tinctoriale comprenant dans un milieu cosmétique approprié, au moins un colorant fluorescent de formule (I) ou (II) tel que défini dans une quelconque des revendications 1 à 9 et au moins un agent réducteur.

**12.** Procédé de coloration de matières kératiniques, dans lequel on applique sur les matières, une composition tinctoriale, comprenant dans un milieu cosmétique approprié, au moins un colorant, fluorescent de formule (I) ou (II) tel que défini dans une quelconque des revendications 1 à 9, éventuellement en présence d'un agent réducteur capable de réduire les liaisons disulfures des matières kératiniques.

**13.** Procédé de coloration de matières kératiniques selon la revendication précédente **caractérisé en ce que** les matières kératiniques sont des fibres kératiniques foncées de hauteur de ton inférieure ou égale à 6.

**14.** Procédé selon l'une quelconque des revendications 12 ou 13, dans lequel le colorant fluorescent de formule (I) ou (II) est présent en quantité comprise entre 0,001 et 50% en poids par rapport au poids total de la composition.

**15.** Dispositif à plusieurs compartiments dans lequel un premier compartiment contient une composition tinctoriale comprenant un colorant fluorescent tel que défini aux revendications 1 à 9 et un deuxième compartiment contient un agent réducteur capable de réduire les liaisons disulfures des matières kératiniques.

**16.** Utilisation des colorants fluorescents tels que définis aux revendications 1 à 9 pour l'éclaircissement des fibres kératiniques foncées de hauteur de ton inférieure ou égale à 6.

## Claims

**1.** Fluorescent dye of formula (I) or (II) below:

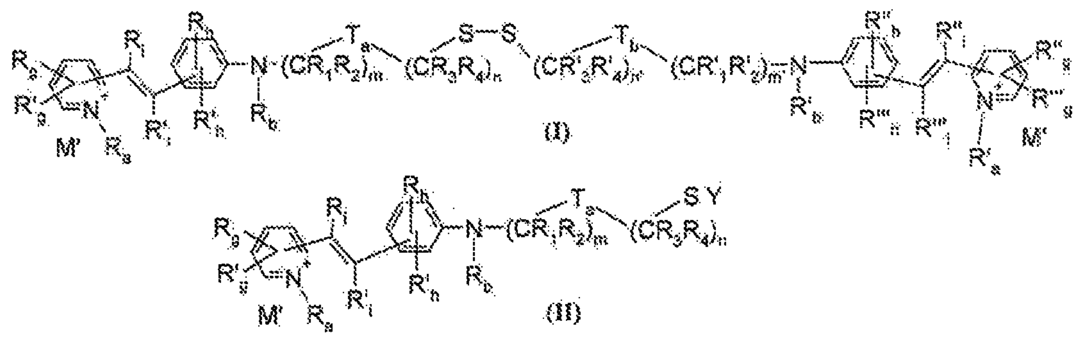

(I)

(II)

the organic or mineral acid salts, optical isomers and geometrical isomers thereof, and the solvates such as hydrates; in which formulae (I) and (II):

> ➢ $R_a$ and $R'_a$, which may be identical or different, represent an aryl($C_1$-$C_4$)alkyl group or a ($C_1$-$C_6$)alkyl group optionally substituted with a hydroxyl or amino, $C_1$-$C_4$ alkylamino or ($C_1$-$C_4$)dialkylamino group; it being possible for said alkyl radicals to form, with the nitrogen atom wich bears them, a heterocycle comprising from 5 to 7 members, optionally comprising another heteroatom which may or may not be different from nitrogen;
> ➢ $R_b$ and $R'_b$, which may be identical or different, represent a hydrogen atom, an aryl ($C_1$-$C_4$)alkyl group or a ($C_1$-$C_6$)alkyl group which is optionally substituted;
> ➢ $R_g$, $R'_g$, $R''_g$ and $R'''_g$, which may be identical or different, represent a hydrogen atom, an amino, ($C_1$-$C_4$) alkylamino, ($C_1$-$C_4$)dialkylamino or trifluoromethyl group, an acylamino, ($C_1$-$C_4$)alkoxy ($C_1$-$C_4$)-alkylcarbonyloxy, ($C_1$-$C_4$)alkoxycarbonyl, ($C_1$-$C_4$)alkyl-carbonylamino or ($C_1$-$C_4$)alkylsulphonylamino radical or a ($C_1$-$C_4$) alkyl radical;
> ➢ $R_h$, $R'_h$, $R''_h$ and $R'''_h$, which may be identical or different, represent a hydrogen atom, a halogen atom, a ($C_1$-$C_4$)dialkylamino or ($C_1$-$C_4$)alkylcarbonyl-amino group, an acylamino or ($C_1$-$C_4$)alkylsulphonylamino radical, or a ($C_1$-$C_4$)alkyl radical;
> ➢ or else two groups $R_g$ and $R'_g$; $R''_g$ and $R'''_g$; $R_h$ and $R'_h$; $R''_h$ and $R'''_h$, borne by two adjacent carbon atoms, together form a benzo or indeno ring, or a fused heterocycloalkyl or fused heteroaryl group; the benzo, indeno, heterocycloalkyl or heteroaryl ring being optionally substituted with a halogen atom, an amino, ($C_1$-$C_4$)alkylamino, ($C_1$-$C_4$)dialkylamino, cyano, carboxyl, hydroxyl, trifluoromethyl, acylamino, $C_1$-$C_4$ alkoxy, $C_2$-$C_{4(}$poly)hydroxyalkoxy, ($C_1$-$C_4$)alkylcarbonyloxy, ($C_1$-$C_4$)alkoxycarbonyl or ($C_1$-$C_4$)alkylcarbonylamino group, an acylamino, carbamoyl or ($C_1$-$C_4$)alkylsulphonylamino radical, an aminosulphonyl radical, or a ($C_1$-$C_{16}$)alkyl radical optionally substituted with a group chosen from ($C_1$-$C_{12}$)alkoxy, hydroxyl, cyano, carboxyl, amino, ($C_2$-$C_4$) alkylamino and ($C_1$-$C_4$)dialkylamino, or else the two alkyl radicals borne by the nitrogen atom of the amino group form a heterocycle comprising from 5 to 7 members and optionally comprising another heteroatom identical to or different from that of the nitrogen atom;
> ➢ $R_i$, $R'_i$, $R''_i$ and $R'''_i$, which may be identical or different, represent a hydrogen atom or a ($C_1$-$C_4$)alkyl group;
> ➢ $R_1$, $R_2$, $R_3$, $R_4$, $R'_1$, $R'_2$, $R'_3$ and $R'_4$, which may be identical or different, represent a hydrogen atom or a ($C_1$-$C_4$)alkyl, ($C_1$-$C_4$)alkoxy, hydroxyl, cyano, carboxyl or (di) ($C_1$-$C_4$)(alkyl) amino group, it being possible for said alkyl radical to form, with the nitrogen atom which bears them, a heterocycle comprising from 5 to 7 members, optionally comprising another heteroatom which may or may not be different from nitrogen;
> ➢ $T_a$ and $T_b$, which may be identical or different, represent the combination of radicals chosen from $-SO_2-$, -O-, -S-, -N(R)-, -CO-, with R representing a hydrogen atom, a $C_1$-$C_4$ alkyl or $C_1$-$C_4$ hydroxyalkyl radical; or an aryl ($C_1$-$C_4$)alkyl;
> ➢ m, m', n and n', which may be identical or different, represent an integer between 0 and 6 inclusive, with m+n and m'+n', which may be identical or different, representing an integer between 1 and 10 inclusive;
> ➢ M' represents an anionic counterion; and
> ➢ Y represents: i) a hydrogen atom; ii) an alkali metal; iii) an alkaline earth metal; iv) an ammonium group: $NR^{\alpha}R^{\beta}R^{\gamma}R^{\delta}$ or a phosphonium group: $PR^{\alpha}R^{\beta}R^{\gamma}R^{\delta}$ with $R^{\alpha}$, $R^{\beta}$, $R^{\gamma}$ and $R^{\delta}$, which may be identical or different, representing a hydrogen atom or a ($C_1$-$C_4$) alkyl group; or v) a thiol-function-protecting group;

it being understood that, when the compound of formula (I) or (II) contains other cationic parts, it is associated with one or more anionic counterions allowing formule (I) or (II) to achieve electroneutrality.

**2.** Fluorescent dye (I) or (II) according to Claim 1, in which when $T_a$ and $T_b$ represent -C(O)-N(R)- or -N(R)-C(O)-.

**3.** Fluorescent dye of formula (II) according to either of Claims 1 and 2, in which represents a hydrogen atom or an alkali metal.

**4.** Fluorescent dye of formula (II) according to one of Claims 1 and 2, in which Y represents a protecting group.

**5.** Fluorescent dye of formula (I) according to the preceding claim, in which Y represents a protecting group chosen from the following radicals:

- $(C_1-C_4)$ alkylcarbonyl;
- $(C_1-C_4)$ alkylthiocarbonyl;
- $(C_1-C_4)$ alkoxycarbonyl;
- $(C_1-C_4)$ alkoxythiocarbonyl;
- $(C_1-C_4)$ alkylthiothiocarbonyl;
- (di) $(C_1-C_4)$(alkyl) aminocarbonyl;
- (di) $(C_1-C_4)$(alkyl) aminothiocarbonyl;
- arylcarbonyl;
- aryloxycarbonyl;
- aryl $(C_1-C_4)$alkoxycarbonyl;
- (di) $(C_1-C_4)$(alkyl)aminocarbonyl;
- $(C_1-C_4)$(alkyl)aminocarbonyl;
- carboxyl;
- $SO_3$; $M^+$ with $M^+$ representing an alkali metal or else M' of formula (II) and $M^+$ are absent;
- optionally substituted aryl;
- optionally substituted heteroaryl;
- optionally cationic, optionally substituted heterocycloalkyl;
- isothiouronium $-C(NR'^cR'^d)=NR'^eR'^f$; An with $R'^c$, $R'^d$, $R'^e$ and $R'^f$, which may be identical or different, representing a hydrogen atom or an alkyl group; preferably, $R'^e$ to $R'^f$ represent a hydrogen atom; and An represents an anionic counterion;
- isothiourea $-C(NR'^cR'^d)=NR'^e$; with $R'^c$, $R'^d$ and $R'^s$ as defined above;
- optionally substituted (di)arylalkyl;
- optionally substituted (di) heteroarylalkyl;
- $CR'^1R'^2R'^3$ with $R'^1$, $R'^2$ and $R'^3$, which may be identical or different, representing a halogen atom or a group chosen from:
  - alkyl;
  - alkoxy;
  - optionally substituted aryl;
  - optionally substituted heteroaryl;
  - $P(Z^1)R''^1R''^2R''^3$ with $R''^1$ and $R''^2$, which may be identical or different, representing a hydroxyl, alkoxy or alkyl group, $R''^3$ representing a hydroxyl or $(C_1-C_4)$alkoxy group and $Z^1$ representing an oxygen or sulphur atom;
- a sterically hindered cyclic group; and
- optionally substituted alkoxyalkyl.

**6.** Fluorescent dye according to any one of the preceding claims, in which Y represents an alkali metal or a protecting group chosen from:

➢ $(C_1-C_4)$alkylcarbonyl;
➢ arylcarbonyl ;
➢ $(C_1-C_4)$alkoxycarbonyl;
➢ aryloxycarbonyl;
➢ aryl $(C_1-C_4)$alkoxycarbonyl;
➢ (di)$(C_1-C_4)$(alkyl)aminocarbonyl;
➢ $(C_1-C_4)$(alkyl)arylaminocarbonyl;
➢ optionally substituted aryl;
➢ 5- or 6-membered monocyclic heteroaryl;
➢ 8- to 11-membered cationic bicyclic heteroaryl such as benzoimidazolium or benzoxazolium; these groups

being optionally substituted with one or more ($C_1$-$C_4$)alkyl groups, which may be identical or different;

➢ cationic heterocycle of formula below:

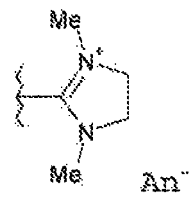

➢ isothiouromium $-C(NH_2)=N^+H_2$; $An^-$;

➢ isothiourea $-C(NH_2)=NH$; and

➢ $SO_3^-$, $M^+$ with $M^+$ representing an alkali metal or else M' of formula (II) and $M^+$ are absent.

**7.** Fluorescent dye according to any one of the preceding claims, chosen from fluorescent dyes of formula **(Ia)** or **(IIa)**, each having an ethylene group linking the pyridinium part to the phenyl at the ortho- or para-position, i.e. at the 2-4', 4-2', 4-4' positions:

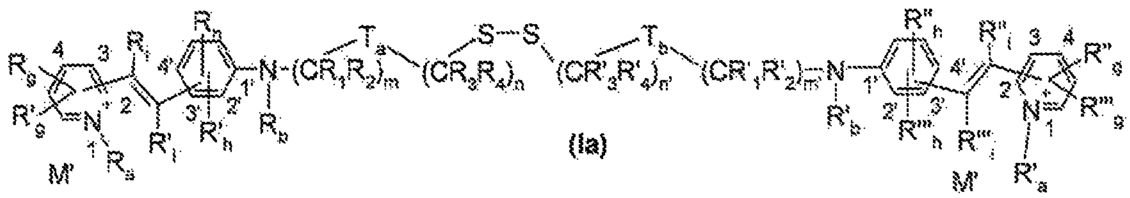

(Ia)

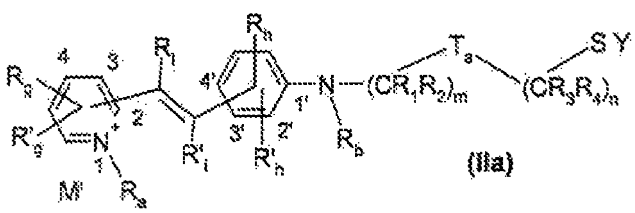

(IIa)

formulae (Ia) and (IIa) with $R_1$, $R_2$, $R_3$, $R_4$, $R_a$, $R_b$, $R_i$, $R'_i$, $R_g$, $R'_g$, $R_h$, $R'_h$, m, n, Y and $R'_1$, $R'_2$, $R'_3$, $R'_4$, $R'_a$, $R'_b$, $R''_i$, $R'''_i$, $R''_g$, $R'''_g$, $R''_h$, $R'''_h$, m' and n' as defined in any one of the preceding claims.

**8.** Fluorescent dye of formula (I) or (Ia) according to either of Claims 1 and 7, which is symmetrical.

**9.** Fluorescent dye according to any one of the preceding claims, chosen from the following dyes:

| | |
|---|---|
| 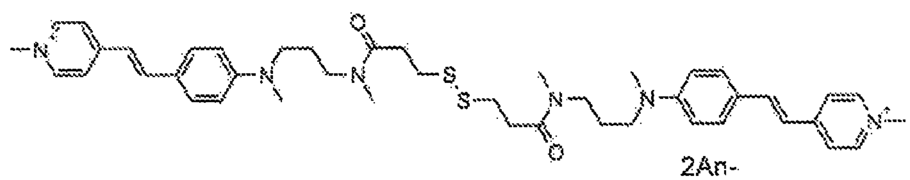 | |
| 1 | |
| 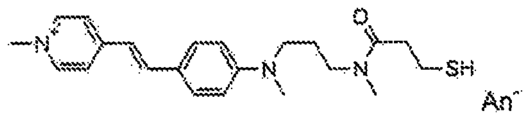 | 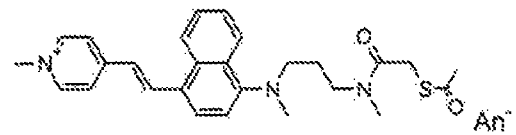 |
| 2 | 3 |
| 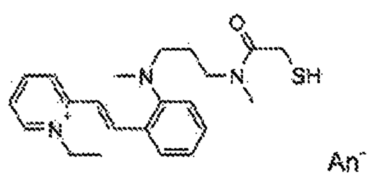 | 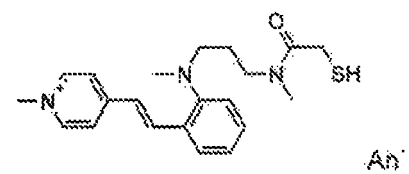 |
| 3 | 4 |
| 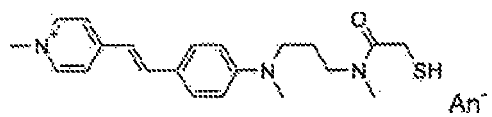 | 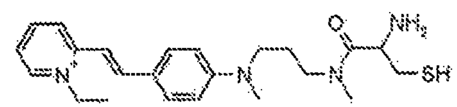 6 |
| 5 | |
| 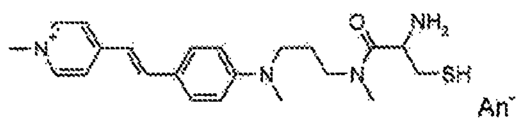 | 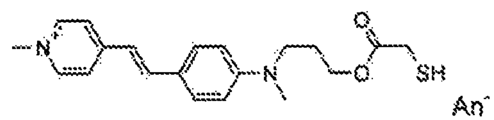 |
| 7 | 8 |

(continued)

| | |
|---|---|
| 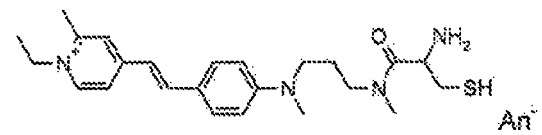 | 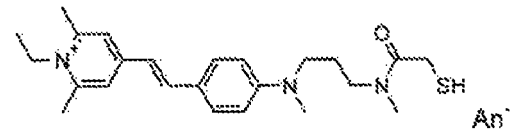 |
| 9 | 10 |
| 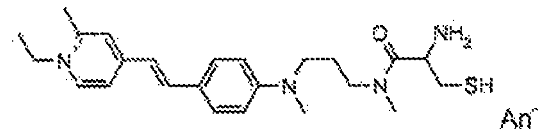 | 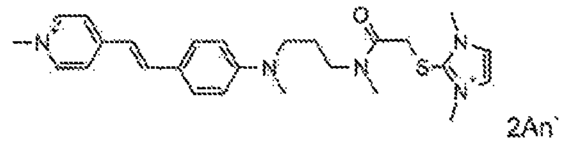 |
| 11 | 12 |
| | 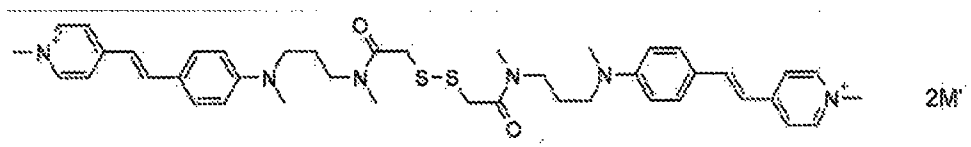 |
| 13 | |
| 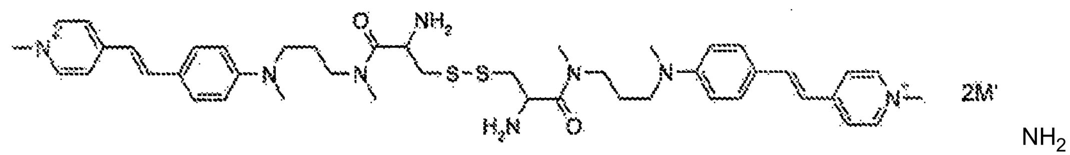 | |
| 14 | |
| 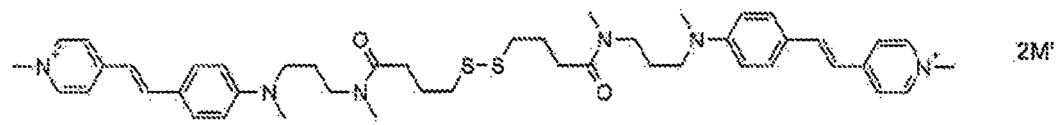 | |
| 15 | |

(continued)

| |
|---|
| 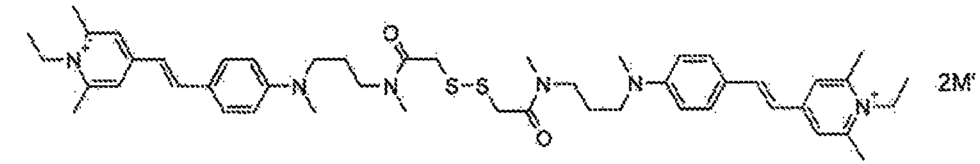 |
| 16 |
| 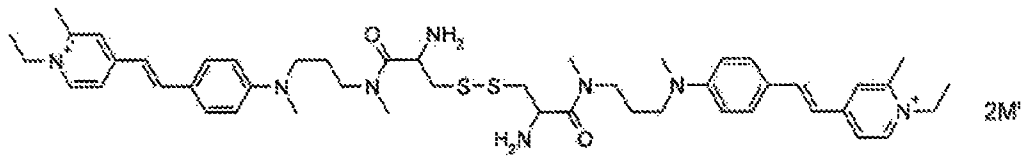 |
| 17 |
| 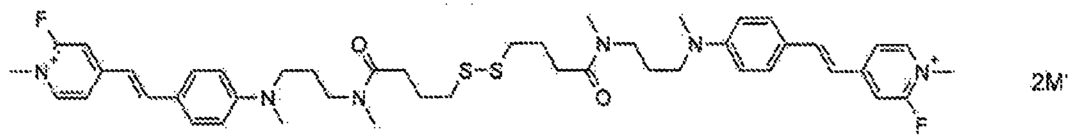 |
| 18 |
| 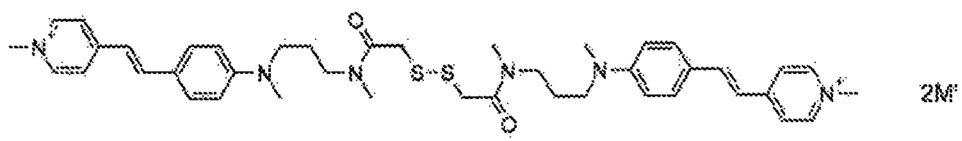 |
| 19 |
| 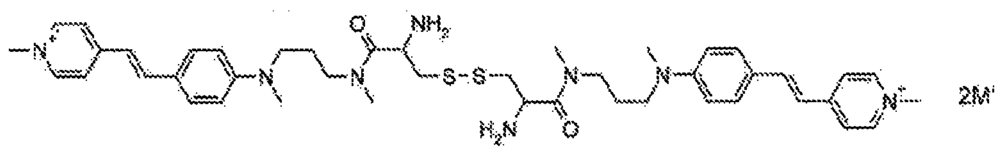 |
| 20 |

(continued)

| |
|---|
| 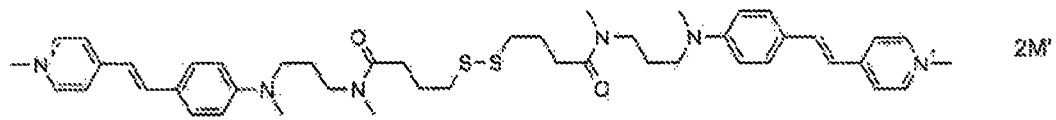 |
| **21** |
| 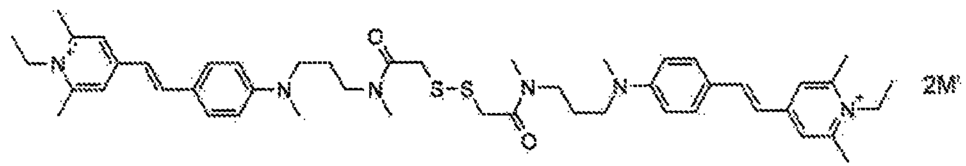 |
| **22** |
| 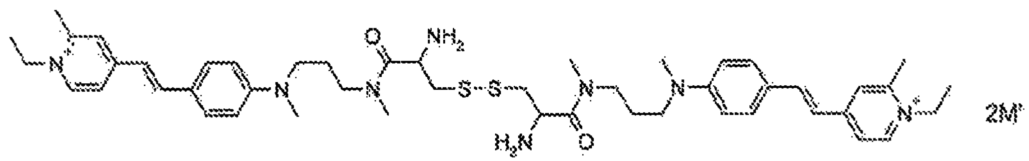 |
| **23** |
| 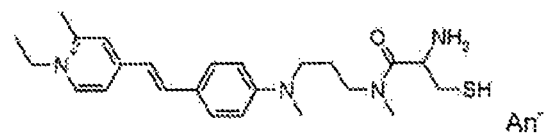 |
| **24** |
| 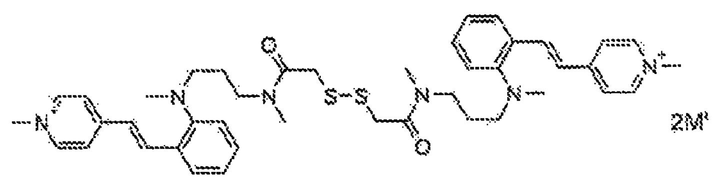 |
| **25** |

(continued)

| |
|---|
| 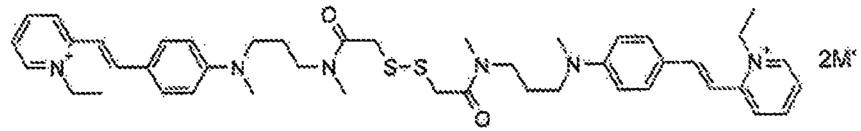 |
| **26** |
| 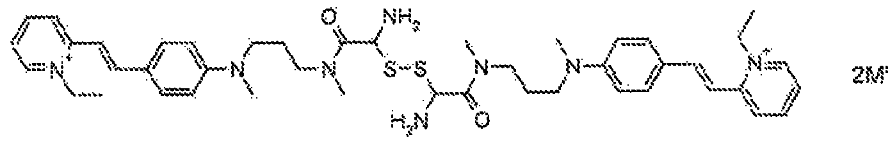 |
| **27** |
| 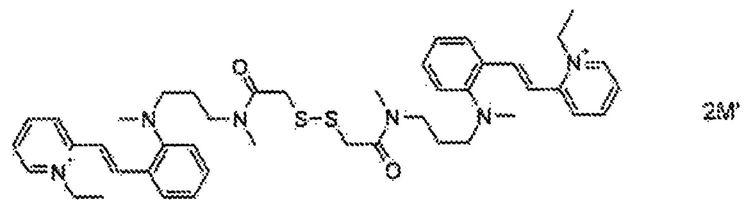 |
| **28** |

with M', $An^-$, which may be identical or different, representing an anionic counterion.

**10.** Dye composition comprising, in a suitable cosmetic medium, a fluorescent, dye of formula (I) or (II) as defined in any one of Claims 1 to 9.

**11.** Dye composition comprising, in a suitable cosmetic medium, at least one fluorescent dye of formula (I) or (II) as defined in any one of Claims 1 to 9 and as least one reducing agent.

**12.** Process for dyeing keratin materials, in which a dye composition, comprising, is a suitable cosmetic medium, at least one fluorescent dye of formula **(I)** or **(II)** as defined in any one of Claims 1 to 9, optionally in the presence of a reducing agent capable of reducing the disulphide bonds of the keratin materials, is applied to the materials.

**13.** Process for dyeing keratin materials according to the preceding claim, **characterized in** the keratin materials are dark keratin fibres having a tone height of less than or equal to 6.

**14.** Process according to either of Claims 12 and 13, in which the fluorescent dye of formula (I) or (II) is present in an amount of between 0.001% and 50% by weight relative to the total weight of the composition.

**15.** Multicompartment device in which a first compartment contains a dye composition comprising a fluorescent dye as defined in Claims 1 to 9 and a second compartment contains a reducing agent capable of reducing the disulphide bonds of the keratin materials.

**16.** Use of the fluorescent dyes as defined in Claims 1 to 9, for lightening dark keratin fibres having a tone height, of less than or equal to 6.

**Patentansprüche**

**1.** Fluoreszenzfarbstoff der folgenden Formel (I) oder (II):

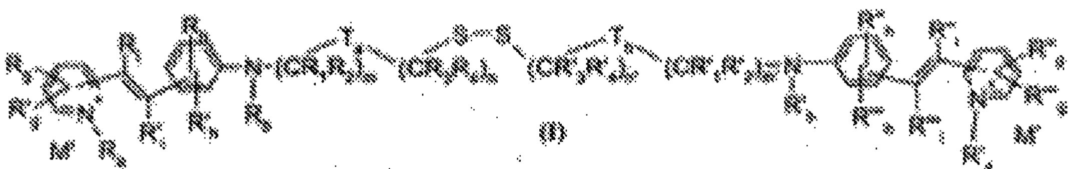

(I)

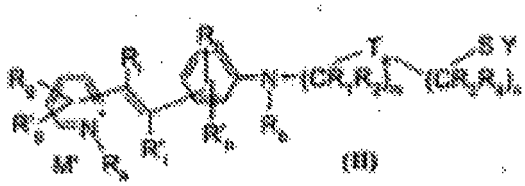

(II)

seine Salze mit einer organischen oder anorganischen Säure, optischen Isomere, geometrischen Isomere und Solvate wie Hydrate;
in der Formel (I) oder (II):

- ➢ die Gruppen $R_a$ und $R'_a$, die gleich oder verschieden sind, bedeuten eine Aryl($C_{1-4}$)alkylgruppe oder eine ($C_{1-6}$)Alkylgruppe, die gegebenenfalls substituiert ist mit Hydroxy oder Amino, ($C_{1-4}$)Alkylamino oder ($C_{1-4}$) Dialhylamino, wobei die Alkylgruppen mit dem Stickstoffatom, von dem sie getragen werden, einen 5- bis 7-gliedrigen Heterocyclus bilden können, der gegebenenfalls ein weiteres Heteroatom enthält, das mit dem Stickstoffatom übereinstimmt oder davon verschieden ist;
- ➢ die Gruppen $R_b$ und $R'_b$, die gleich oder verschieden sind, bedeuten ein Wasserstoffatom, eine Aryl($C_{1-4}$) alkylgruppe oder eine ($C_{1-6}$)Alkylgruppe, die gegebenenfalls substituiert ist;
- ➢ die Gruppen $R_g$, $R'_g$, $R''_g$ und $R'''_g$, die gleich oder verschieden sind, bedeuten ein Wasserstoffatom, eine Aminogruppe, eine ($C_{1-4}$)Alkylaminogruppe, eine ($C_{1-4}$)Dialkylaminogruppe, eine Trifluormethylgruppe, eine Acylaminogruppe, eine ($C_{1-4}$)-Alkoxygruppe, eine ($C_{1-4}$)Alkylcarbonyloxygruppe, eine ($C_{1-4}$)Alkoxycarbonylgruppe, eine ($C_{1-4}$)Alkylcarbonylaminogruppe, eine ($C_{1-4}$)Alkylsulfonylaminogruppe, eine ($C_{1-3}$)-Alkygruppe;

➢ die Gruppen $R_h$, $R'_h$, $R''_h$ und $R'''_h$, die gleich oder verschieden sind, bedeuten ein Wasserstoffatom, ein Halogenatom, eine $(C_{1-4})$Dialkylammogruppe, eine $(C_{1-4})$Alkylcarbonylaminogruppe, eine Acylaminogruppe, eine $(C_{1-4})$Alkylsulfonylaminogruppe oder eine $(C_{1-4})$Alkylgruppe;

➢ oder zwei Gruppen $R_g$ und $R'_g$; $R''_g$ und $R'''_g$; $R_h$ und $R'_h$; $R''_h$ und $R'''_h$, die von zwei benachbarten Kohlenstoffatomen getragen werden, bilden gemeinsam einen Benzoring, einen Indenoring, eine kondensierte Heterocycloalkylgruppe oder eine kondensierte Heteroarylgruppe; wobei der Benzoring, der Indenoring, die Heterocyclolkylgruppe und die Heteroarylgruppe gegebenenfalls substituiert sind mit Halogen, Amino, $(C_{1-4})$Alkylamino, $(C_{1-4})$Dialkylamino, Cyano, Carboxy, Hydroxy, Trifluormethyl, Acylamino, $(C_{1-4})$Alkoxy, $(C_{2-4})$(Poly)-hydroxyalkoxy, $(C_{1-4})$Alkylcarbonyloxy, $(C_{1-4})$Alkoxycarbonyl, $(C_{1-4})$Alkylcarbonylamino, Acylamino, Carbamoyl, $(C_{1-4})$Alkylsulfonylamino, Aminosulfonyl oder $(C_{1-16})$Alkyl, wobei diese Gruppe gegebenenfalls substituiert ist mit einer Gruppe, die ausgewählt ist unter $(C_{1-12})$Alkoxy hydroxy, Cyano, Carboxy, Amino, $(C_{1-4})$Alkylamino und $(C_{1-4})$Dialkylamino, wobei die beiden Alkylgruppen, die von dem Stickstoffatom der Aminogruppe getragen werden, einen 5- bis 7-gliedrigen Heterocyclus bilden, der gegebenenfalls ein weiteres Heteroatom enthält, das mit dem Stickstoffatom übereinstimmt oder davon verschieden ist;

➢ die Gruppen $R_i$, $R'_i$, $R''_i$ und $R'''_i$, die gleich oder verschieden sind, bedeuten ein Wasserstoffatom oder eine $(C_{1-4})$Alkylgruppe;

➢ die Gruppen $R_1$, $R_2$, $R_3$, $R_4$, $R'_1$, $R'_2$, $R'_3$ und $R'_4$, die gleich oder verschieden sind, bedeuten ein Wasserstoffatom oder eine Gruppe $(C_{1-4})$Alkyl, $(C_{1-12})$Alkoxy, Hydroxy, Cyano, Carboxy, $(C_{1-4})$Dialkylamino, wobei die Alkylgruppen mit dem Strickstoffatom, von dem sie getragen werden, einen 5- bis 7-gliedrigen Heterocyclus bilden der gegebenenfalls ein weiteres Heteroatom enthält, das mit dem Stickstoffatom übereinstimmt oder davon verschieden ist;

➢ die Gruppen $T_a$ und $T_b$, die gleich oder verschieden sind, bedeuten Kombinationen von Gruppen, die ausgewählt sind unter: $-SO_2-$, -O-, -S-, -N(R)-, -C(O)-, wobei R ein Wasserstoffatom, eine $(C_{1-4})$Alkylgruppe, eine $(C_{1-4})$Hydroxyalkylgruppe oder eine Aryl$(C_{1-4})$alkylgruppe bedeutet;

➢ m, m', n und n', die gleich oder verschieden sind, bedeuten eine ganze Zahl im Bereich von 0 bis 6 (Grenzen eingeschlossen) mit m + n und m' + n', die gleich oder verschieden sind, bedeuten eine ganze Zahl im Bereich von 1 bis 10 (Grenzen eingeschlossen);

➢ M' ist ein anionisches Gegenion; und

➢ Y bedeutet: i) ein Wasserstoffatom, ii) ein Alkalimetall; iii) ein Erdalkalimetall; iv) eine Ammoniumgruppe: $N^+R^\alpha R^\beta R^\gamma R^\delta$ oder eine Phosphoniumgruppe: $P^+R^\alpha R^\beta R^\gamma R^\delta$, wobei $R^\alpha$, $R^\beta$, $R^\gamma$ und $R^\delta$, die gleich oder verschieden sind, ein Wasserstoffatom oder eine $(C_{1-4})$Alkylgruppe bedeuten; oder v) eine Schutzgruppe für die Thiofunktion;

mit der Meßgabe, dass:

- wenn die Verbindung der Formel (I) oder (II) weitere kationische Teile sie mit einem oder mehreren anionischen Gegenionen kombiniert ist, mit denen die Elektroneutralität der Formeln (I) oder (II) erreicht werden kann.

**2.** Fluoreszierender Farbstoff der Formel (I) oder (II) nach Anspruch 1, worin $T_a$ und $T_b$ -C(O)-N(R)- oder -N(R)-C(O)- bedeuten.

**3.** Fluoressierender Farbstoff der Formel (II) nach Anspruch 1 oder 2, bei dem Y ein Wasserstoffatom oder ein Alkalimetall bedeutet.

**4.** Fluoreszierender Farbstoff der Formel (II) nach Anspruch 1 oder 2, bei dem Y eine Schutzgruppe bedeutet.

**5.** Fluoreszierender Farbstoff der Formel (I) nach dem vorhergehenden Anspruch, bei dem Y eine Schutzgruppe bedeutet, die unter den folgenden Gruppen ausgewählt ist:

➢ $(C_{1-4})$Alkylcarbonyl;
➢ $(C_{1-4})$Alkyithiocarbonyl;
➢ $(C_{1-4})$Alkoxycarbonyl;
➢ $(C_{1-4})$Alkoxythiocarbonyl;
➢ $(C_{1-4})$Alkylthio-thiocarbonyl;
➢ (Di)$(C_{1-4})$(alkyl)aminocarbonyl;
➢ (Di)$(C_{1-4})$(alkyl)aminothiocarbonyl;
➢ Arylcarbonyl;
➢ Aryloxycarbonyl;
➢ Aryl$(C_{1-4})$alkoxycarbonyl;

➢ (Di)($C_{1-4}$)alkyl)aminocarbonyl;
➢ ($C_{1-4}$)(Alkyl)arylaminocarbonyl;
➢ Carboxy;
➢ $SO_3$;$M^+$ mit $M^+$ bedeutet ein Alkalimetall oder $M^+$ der Formel (II) und $M^+$ fehlen;
➢ Aryrl, gegebenenfalls substituiert;
➢ Hekeroaryl, gegebenenfalls substituiert;
➢ Heterocycloalkyl, gegebenenfalls substituiert;
➢ Heterocycloalkyl, gegebenenfalls substituiert, gegebenenfalls kationisch;
➢ Isothiouronium -C(NR'$^c$R'$^d$)=N$^+$R'$^e$R'$^f$; An; wobei R'$^c$, R'$^d$, R'$^c$ und R'$^f$, die gleich oder verschieden sind, ein Wasserstoffatom oder eine Alkylgruppe bedeuten; wobei R'$^c$ bis R'$^f$ vorzugsweise ein Wasserstoffatom bedeuten; und An ein anionisches Gegenion ist;
➢ Isothioharnstoff -C(NR'$^c$R'$^c$=NR'$^c$ wobei R'$^c$, R'$^d$ und R'$^e$ die oben angegebenen Bedeutungen ausweisen;
➢ (Di)arylalkyl, gegebenenfalls substituiert;
➢ (Di)heteroarylalkyl, gegebenenfalls substituiert;
➢ CR'$^1$R'$^2$R'$^3$, wobei R'$^1$, R'$^2$ und R'$^3$, die gleich oder verschieden sind, ein Halogenatom oder eine Gruppe bedeuten, die ausgewählt ist unter:

- Alkyl;
- Alkoxy;
- Aryl, gegebenenfalls substituiert,
- Heteroaryl, gegebenenfalls substituiert;

➢ P($Z^1$)R"$^1$R"$^2$R"$^3$, wobei R"$^1$ und R"$^2$, die gleich oder verschieden sind, Hydroxy, Alkoxy oder Alkyl bedeuten, R"$^3$ Hydroxy oder Alkoxy ist und $Z^1$ ein Sauerstoffatom oder ein Schwefelatom bedeutet;
➢ cyclisch sterisch gehindert; und
➢ Alkoxyalkyl, gegebenenfalls substituiert,

**6.** Fluoreszierender Farbstoff nach einem der vorhergehenden Ansprüche, bei dem Y ein Alkalimetall oder eine Schutzgruppe bedeutet, die ausgewählt ist unter:

♦ ($C_{1-4}$)Alkylcarbonyl;
♦ Arylcarbonyl;
♦ ($C_{1-4}$)Alkoxycarbonyl;
♦ Aryloxycarbonyl;
♦ Aryl($C_{1-4}$)alkoxycarbonyl;
♦ (Di)($C_{1-4}$alkyl)aminocarbonyl;
♦ ($C_{1-4}$)(Alkyl)arylaminocarbonyl;
♦ Aryl, gegebenenfalls substituiert;
♦ 5- oder 6-gliedriges monocyclisches Heteroaryl;
♦ 8- bis 11-gliedriges kationisches bicyclisches Heteroaryl, wie Benzoimidazolium oder Benzoxazolium, wobei diese Gruppen gegebenenfalls mit einer oder mehreren, gleichen oder verschiedenen ($C_{1-4}$)Alkylgruppen substituiert sein können;
♦ dem kationischen Heterocyclus der folgenden Formel:

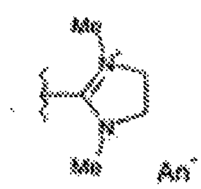

♦ Isothiouronium -C($NH_2$)=$N^+H_2$, An-;
♦ Isothioharnstoff -C($NH_2$)=NH; und
♦ $SO_3^-$, $M^+$ mit $M^+$ bedeutet ein Alkalimetall oder $M^+$ der Formel (II) und $M^+$ fehlen.

**7.** Fluoreszierender Farbstoff nach einem der vorhergehenden Ansprüche, der unter den fluoreszierenden Farbstoffen der Formel (Ia) oder (IIa) ausgewählt ist, wobei jeder eine Ethylengruppe aufweist, die den Pyridiniumteil mit dem

Phenylteil in ortho- oder para-Stellung verbindet, d.h. den Positionen 4-4', 4-2', oder 2-4':

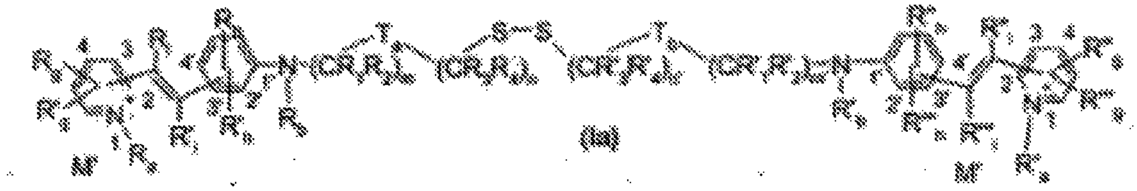

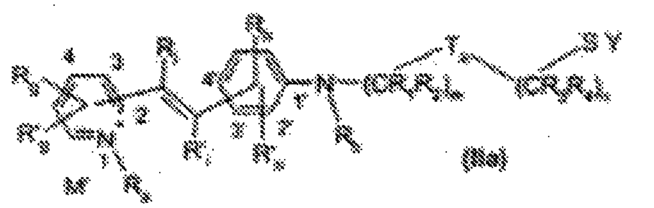

in den Formeln (Ia) oder (IIa) sind die Gruppen $R_1$, $R_2$, $R_3$, $R_4$, $R_a$, $R_b$, $R_c$, $R_d$, $R_i$, $R'_i$, $R_g$, $R'_g$, $R_h$, $R'_h$, m, n, Y und $R'_i$, $R'_2$, $R'_3$, $R'_4$, $R'_a$, $R'_b$, $R'_c$, $R'_d$, $R''_i$, $R'''_i$, $R''_g$, $R'''_g$, $R''_h$, $R'''_h$, m' und n' wie in einem der vorhergehenden Ansprüche definiert.

**8.** Fluoreszierender Farbstoff der Formel (I) oder (Ia) nach einem der Ansprüche 1 oder 7, der symmetrisch ist.

**9.** Fluoreszierender Farbstoff nach einem der vorhergehenden AnSprüche, der unter den folgenden Farbstoffen ausgewählt ist:

| | |
|---|---|
| 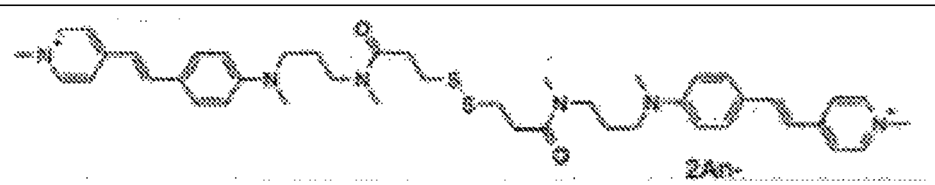 | |
| 1 | |
| 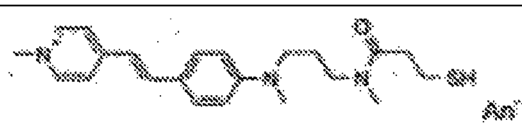 | 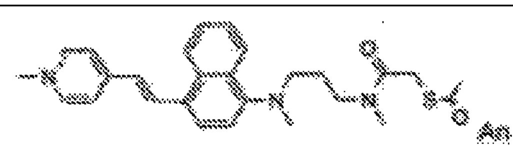 |
| 2 | 3 |
| 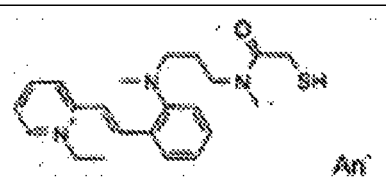 | 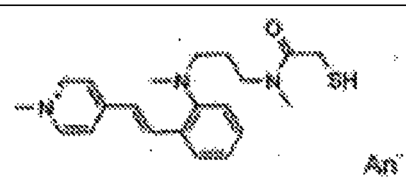 |
| 3 | 4 |
| 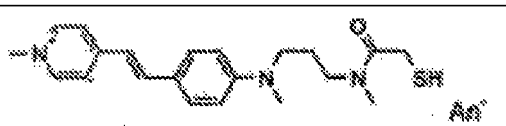 | 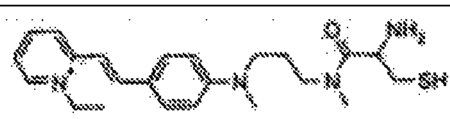 |
| 5 | 6 |

(fortgesetzt)

<table>
<tr><td>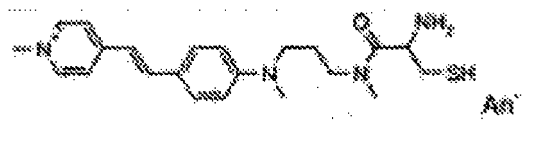</td><td>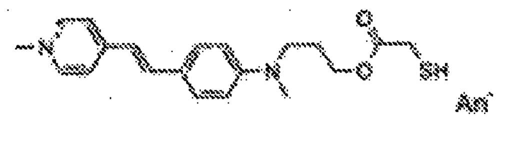</td></tr>
<tr><td>7</td><td>8</td></tr>
<tr><td>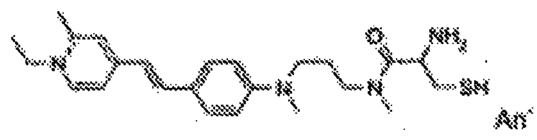</td><td>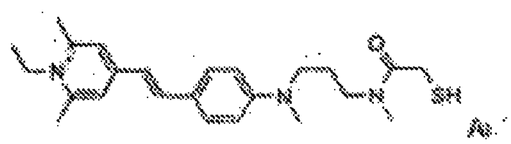</td></tr>
<tr><td>9</td><td>10</td></tr>
<tr><td>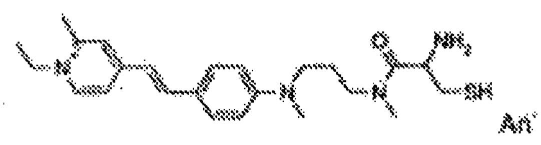</td><td>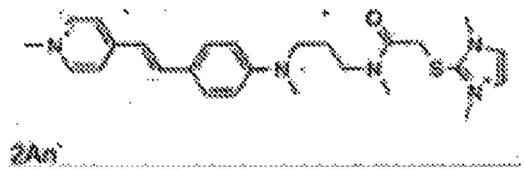</td></tr>
<tr><td>11</td><td>12</td></tr>
<tr><td colspan="2">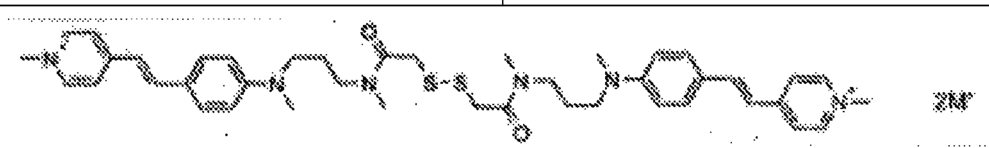</td></tr>
<tr><td colspan="2">13</td></tr>
<tr><td colspan="2">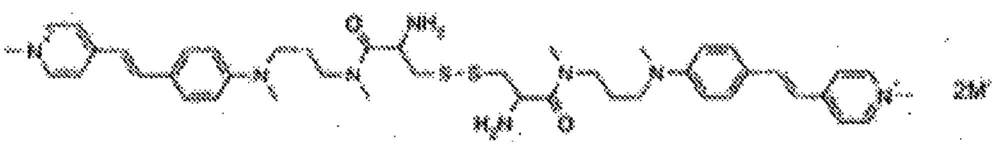</td></tr>
<tr><td colspan="2">14</td></tr>
<tr><td colspan="2">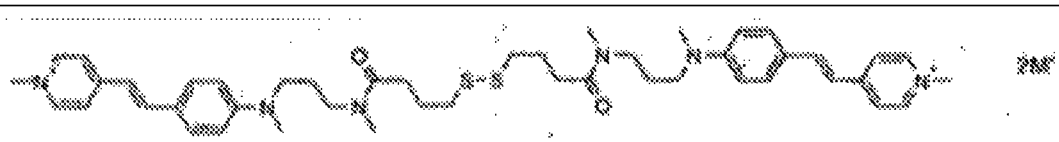</td></tr>
<tr><td colspan="2">15</td></tr>
<tr><td colspan="2">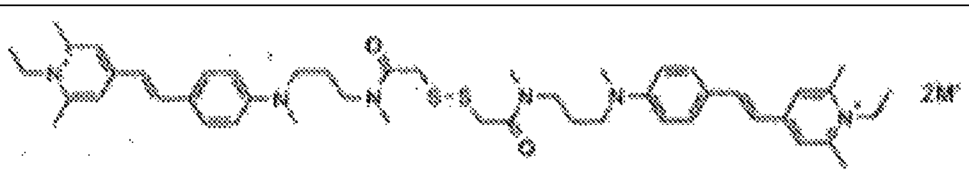</td></tr>
<tr><td colspan="2">16</td></tr>
<tr><td colspan="2">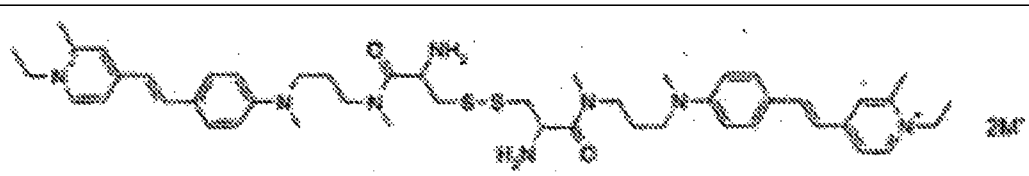</td></tr>
<tr><td colspan="2">17</td></tr>
</table>

(fortgesetzt)

| |
|---|
| 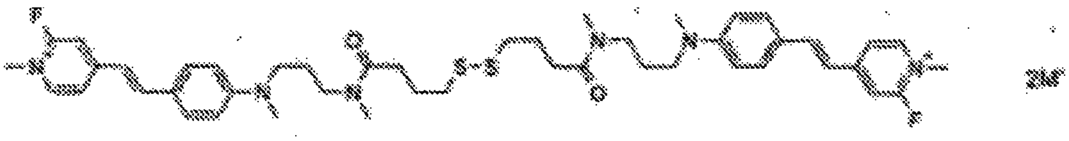 |
| **18** |
| 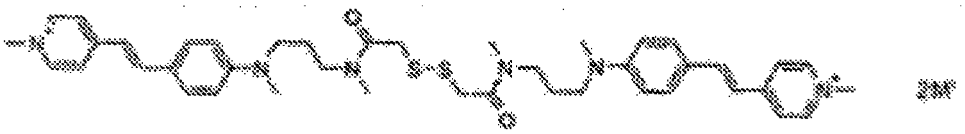 |
| **19** |
| 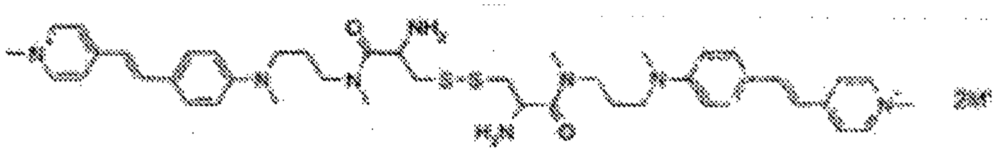 |
| **20** |
| 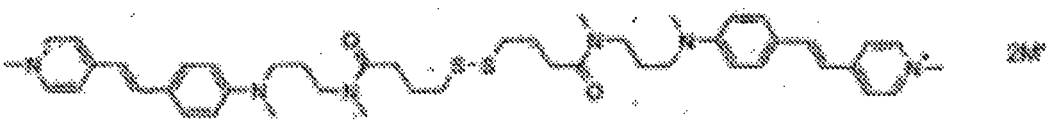 |
| **21** |
| 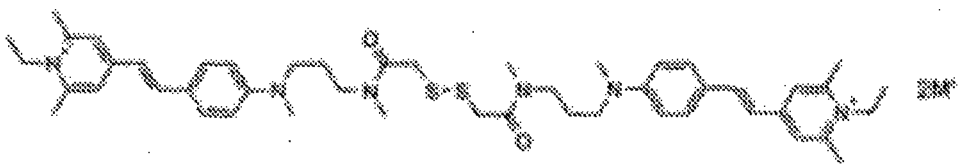 |
| **22** |
| 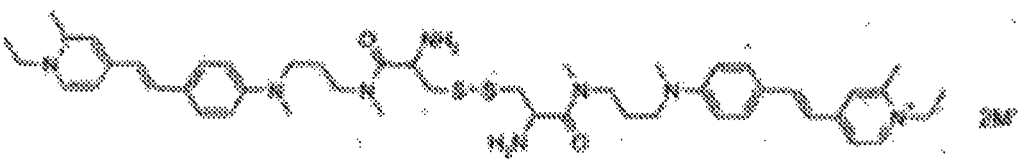 |
| **23** |
| 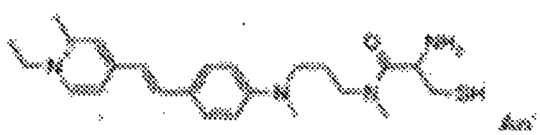 |
| **24** |
| 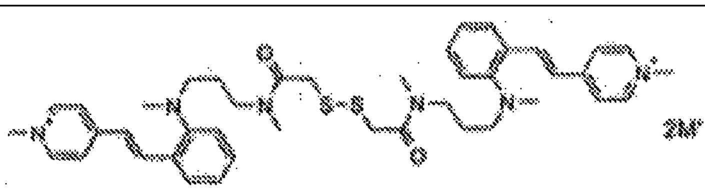 |
| **25** |

(fortgesetzt)

| |
|---|
| 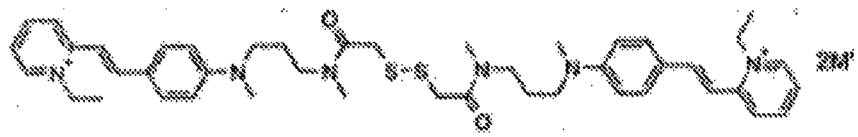 |
| **26** |
| 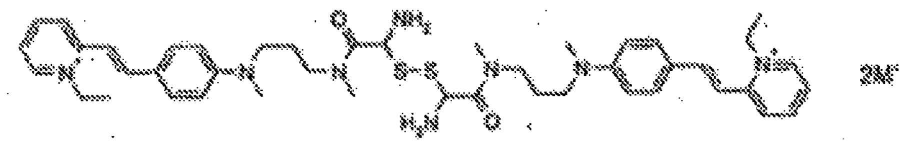 |
| **27** |
| 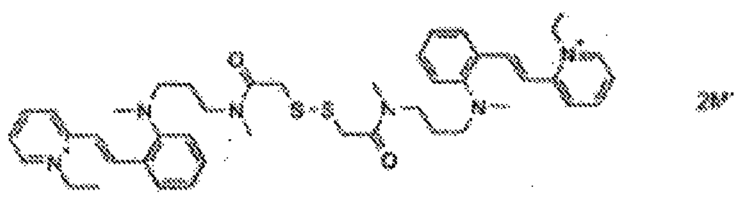 |
| **28** |

wobei M' und An-, die gleich oder verschieden sind, ein anionisches Gegenion bedeuten.

**10.** Farbmittelzusammensetzung, die in einem kosmetisch geeigneten Medium einen fluoreszierenden Farbstoff der Formel (I) oder (II) enthält, wie er in einem der Ansprüche 1 bis 9 definiert ist.

**11.** Farbmittelzusammensetzung, die in einem kosmetisch geeigneten Medium einen fluoreszierenden Farbstoff der Formel (I) oder (II), wie er in einem der Ansprüche 1 bis 9 definiert ist, und mindestens ein Reduktionsmittel enthält,

**12.** Verfahren zum Färben von Keratinsubstanzen, wobei auf die Keratinsubstanzen eine Farbmittelzusammensetzung aufgetragen wird, die in einem kosmetisch geeigneten Medium mindestens einen fluoreszierenden Farbstoff der Formel (I) oder (II), wie er in einem der Ansprüche 1 bis 9 definiert ist, gegebenenfalls in Gegenwart eines Reduktionsmittels enthält, das befähigt ist, die Disulfidbindungen von Keratinsubstanzen zu reduzieren.

**13.** Verfahren zum Färben von Keratinsubstanzen nach dem vorhergehenden Anspruch, **dadurch gekennzeichnet, dass** die Keratinsubstanzen dunkle Keratinfasern sind, die einen Farbton von 6 oder darunter aufweisen.

**14.** Verfahren nach einem, der Ansprüche 12 oder 13, bei dem der fluoreszierende Farbstoff der Formel (I) oder (II) in einem Mengenanteil von 0,001 bis 50 Gew.-%, bezogen auf das Gesamtgewicht der Zusammensetzung, enthalten ist.

**15.** Vorrichtung mit mehreren Abteilungen, wobei eine erste Abteilung eine Farbmittelzusammensetzung enthält, die einen fluoreszierenden Farbstoff, wie er in einem der Anspruche 1 bis 9 definiert ist, aufweiset, und eine zweite Abteilung ein Reduktionsmittel enthält, das befähigt ist, die Disulfidbindungen von Keratinsubstanzen zu reduzieren.

**16.** Verwendung von fluoreszierenden Farbstoffen, wie sie in einem der Ansprüche 1 bis 9 definiert sind, zum Aufhellen von dunklen Keratinfasern, die einen Farbton von 6 oder darunter aufweisen.

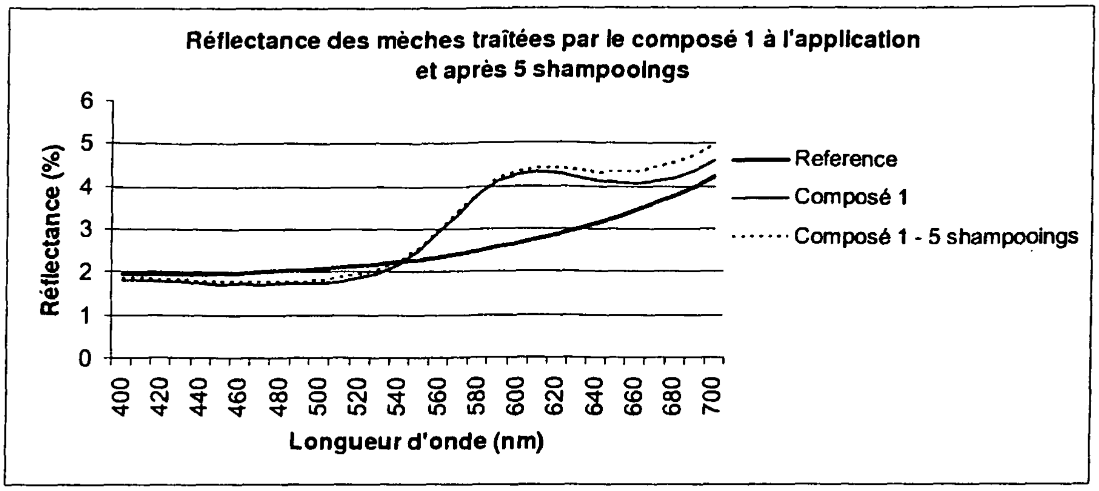
Réflectance des mèches traîtées par le composé 1 à l'application
et après 5 shampooings
Réflectance (%)
6
5
4
3
2
1
0
400
420
440
460
480
500
520
540
560
580
600
620
640
660
680
700
Longueur d'onde (nm)
Reference
Composé 1
Composé 1 - 5 shampooings

fig. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2830189
- WO 2004091473 A
- CA 2024509
- WO 9951194 A
- FR 1156407
- WO 2005097051 A
- DE 19951134
- FR 2586913


### Littérature non-brevet citée dans la description


- **Guise ; Stapleton.** *Journal of the Society of Dyers and Colourists,* 1975, vol. 91, 259-264
- *Journal of Cosmetic Chemistry,* 1991, vol. 42, 1-17
- **Chartes ZVIAK.** Science des traitements capillaires. 1988, 215-278
- **T. W. Greene.** Protective Groups in Organic Synthesis. John Willey & Sons, 1981, 193-217
- **P. Kocienski.** Protecting Groups. Thieme, 2005
- Thiols and organic Sulfides. Ullmann's Encyclopedia. Wiley-VCH, 2005
- Reactions, Mechanisms and Structures. **J. March.** Advanced Organic Chemistry. John Willey & Sons, 1992
- **T. W. Greene.** *Protective Groups in Organic Synthesis*
- **T. W. Greene.** Protective Groups in Organic Synthesis. John Willey & Sons, 1981
- **J. March.** Advanced Organic Chemistry. John Willey & Sons, 1992